(19) Europäisches Patentamt
European Patent Office
Office européen des brevets

(11) Veröffentlichungsnummer: **0 360 131**
**A1**

(12) **EUROPÄISCHE PATENTANMELDUNG**

(21) Anmeldenummer: **89116833.8**

(22) Anmeldetag: **12.09.89**

(51) Int. Cl.5: **C07D 405/04 , A61K 31/40 , A61K 31/44**

Patentansprüche für folgende Vertragsstaaten: ES + GR.

(30) Priorität: **17.09.88 DE 3831697**

(43) Veröffentlichungstag der Anmeldung: **28.03.90 Patentblatt 90/13**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Utz, Roland, Dr.**
**Sudetenstrasse 18**
**D-6101 Messel(DE)**
Erfinder: **Englert, Heinrich Christian, Dr.**
**Stormstrasse 13**
**D-6238 Hofheim am Taunus(DE)**
Erfinder: **Schölkens, Bernward, Dr.**
**Hölderlinstrasse 62**
**D-6233 Kelkheim (Taunus)(DE)**
Erfinder: **Klaus, Erik, Dr.**
**Parkstrasse 12**
**D-6233 Kelkheim (Taunus)(DE)**

(54) **2,3,4,5-Tetrahydro-1-benzoxepine, Verfahren zu ihrer Herstellung, ihre Verwendung sowie pharmazeutische Präparate auf Basis dieser Verbindungen.**

(57) 2,3,4,5-Tetrahydro-1-benzoxepine der Formel I

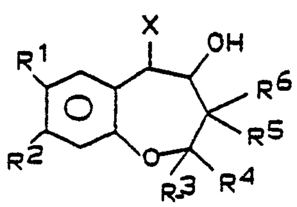

(I),

mit $R^1$ unter anderem gleich H, Alkyl, Alkoxy, Hal, Alkylsulfonyl, Arylsulfonyl, $R^2$ gleich H, Alkyl, Alkoxy, OH, $R^3$ bis $R^6$ H oder Alkyl und X gleich

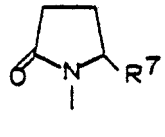

oder

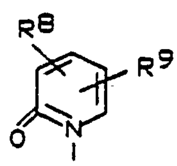

,

haben hervorragende Wirksamkeit als Antihypertensiva, als Coronartherapeutika, als Mittel zur Behandlung der Herzinsuffizienz, der cerebralen bzw. peripheren Durchblutungsstörungen oder von Darmmotilitätsstörungen, vorzeitiger Wehentätigkeit, Obstruktionen der Atemwege oder der ableitenden Harnwege oder der Gallenwege oder als Spasmolytika.

EP 0 360 131 A1

# 2,3,4,5-Tetrahydro-1-benzoxepine, Verfahren zu ihrer Herstellung, ihre Verwendung sowie pharmazeutische Präparate auf Basis dieser Verbindungen

Die Erfindung betrifft 2,3,4,5-Tetrahydro-1-benzoxepine der Formel 1

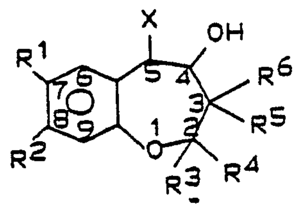
(I),

in welcher

$R^1$ für H, $(C_1-C_4)$-Alkyl, OH, $(C_1-C_4)$-Alkoxy, Halogen, CN, $NO_2$, CO-$(C_1-C_4)$-Alkyl, $-\overset{OH}{\overset{|}{C}}H$ -$(C_1-C_4)$-Alkyl, CO-Ar, $-\overset{OH}{\overset{|}{C}}H$ -Ar, COOH, Carboxy-$(C_1-C_4)$-alkyl, $(C_1-C_4)$-Alkyl-$SO_r$-, $ArSO_r$-, wobei r für 0, 1, 2 und Ar für ein aromatisches oder heteroaromatisches System steht, das unsubstituiert oder durch 1 bis 3 gleiche oder verschiedene Reste $(C_1-C_4)$-Alkyl, $(C_1-C_4)$-Alkoxy, Halogen, CN, $NO_2$ substituiert ist, steht,

$R^2$ für H, OH, $(C_1-C_4)$-Alkoxy, $(C_1-C_4)$-Alkyl steht,

$R^3$,$R^4$,$R^5$,$R^6$ gleich oder verschieden sind und für H oder $(C_1-C_4)$-Alkyl stehen und

X in der Bedeutung von α) oder ß) steht,

α) 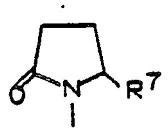

ß) 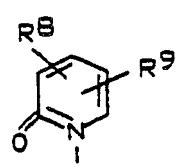
,

wobei $R^7$ H oder Methyl, $R^8$ und $R^9$ gleich oder verschieden sind und für H, $(C_1-C_2)$-Alkyl, Halogen, Nitro oder CN stehen.

Unter einem aromatischen System Ar wird Phenyl, Naphthyl oder Biphenylyl verstanden, ein heteroaromatisches System Ar ist ein Rest eines 5- oder 6-gliedrigen O-, N- und/oder S-heterocyclischen Ringes, insbesondere Furyl, Thienyl, Isothiazolyl, Oxazolyl, Isoxazolyl, Pyrazolyl, Imidazolyl, Thiazolyl, Pyridyl, Pyrazinyl, Pyrimidinyl, Pyridazinyl, Triazinyl.

Unter Halogen wird F, Cl, Br oder J, vorzugsweise F und Cl verstanden.

Die C-Atome 4 und 5 des 2,3,4,5-Tetrahydro-1-benzoxepinsystems der Formel I sind asymmetrisch substituiert. Die Erfindung betrifft dabei nur solche Verbindungen, die an diesen Zentren entgegengesetzte Konfigurationen, also eine "trans"-Orientierung der Substituenten an diesen C-Atomen aufweisen. Enthalten die Substituenten $R^1$, $R^2$, $R^3$, $R^4$, $R^5$, $R^6$ und X Asymmetriezentren oder, falls $R^3/R^4$ und/oder $R^5/R^6$ ungleich sind (und somit ein oder zwei asymmetrische Kohlenstoffatome erzeugen), gehören Verbindungen mit sowohl S- als auch R-konfigurierten Zentren zur Erfindung.

Die Verbindungen können als optische Isomere, als Diastereomere, als Racemate oder als Gemische derselben vorliegen.

Bevorzugt sind Verbindungen der Formel I, bei denen $R^1$ für H, Halogen, CN, Nitro, Phenylsulfinyl, Phenylsulfonyl und Benzoyl steht, wobei die Phenylreste in der obengenannten Bedeutung substituiert sind und $R^2$, $R^3$, $R^4$, $R^5$, $R^6$ und X wie oben definiert sind.

Bevorzugt sind weiterhin Verbindungen der Formel I, bei denen $R^1$ für H, Halogen, CN, Nitro, Phenylsulfinyl, Phenylsulfonyl und Benzoyl steht, wobei die Phenylreste unsubstituiert oder durch 1 bis 2 gleiche oder verschiedene Halogenatome substituiert sind, $R^2$ H bedeutet, $R^3$, $R^4$, $R^5$, $R^6$ und X wie oben definiert sind.

Bevorzugt sind auch Verbindungen der Formel I, bei denen $R^1$ für H, CN, Nitro, Phenylsulfinyl, Phenylsulfonyl und Benzoyl steht, wobei die Phenylreste unsubstituiert oder durch 1 bis 2 gleiche oder

verschiedene Halogenatome substituiert sind, $R^2$, $R^3$, $R^4$, $R^5$ und $R^6$ für ein Wasserstoffatom und X in der Bedeutung von ß steht, wobei $R^8$ und $R^9$ die eingangs erwähnten Definitionen annehmen.

Ganz besonders bevorzugt sind Verbindungen der Formel I, bei denen $R^1$ für H, CN, Nitro, Phenylsulfinyl, Phenylsulfonyl und Benzoyl steht, wobei die Phenylreste unsubstituiert oder durch ein Halogenatom substituiert sind, $R^2$, $R^3$, $R^4$, $R^5$, $R^6$ für ein Wasserstoffatom und X in der Bedeutung von α steht, wobei $R^7$ wie oben definiert ist.

Ähnliche Verbindungen sind bisher nicht bekannt; aus den EP-Offenlegungsschriften 0 277 612, 0 277 611 und 0 273 262 sind Chromansysteme bekannt.

Mit den Verbindungen I wurde nun eine neue Substanzklasse mit wertvollen pharmakologischen Eigenschaften gefunden.

Tierexperimentelle Untersuchungen zeigen, daß sie sich für die Behandlung des gestörten cardiovaskulären Systems eignen, beispielsweise für die Behandlung der Hypertonie, der Herzinsuffizienz oder von Durchblutungsstörungen des Coronarsystems wie etwa Angina. Cerebrale und periphere Durchblutungsstörungen werden ebenfalls günstig beeinflußt. Darüber hinaus können Verbindungen I glattmuskuläre Organe wie Uterus, Bronchien, Darm und Galle, die ableitenden Harnwege (Ureter, Harnblase und Urethra) im Sinne einer Spasmolyse beeinflussen. Sie eignen sich deshalb auch zur Behandlung von Krankheiten, die mit Spasmen dieser Organe verbunden sind, beispielsweise zur Behandlung von vorzeitiger Wehentätigke it bei der Schwangerschaft, von Koliken der Harnleiter oder der Galle, von obstruktiven Atemwegserkrankungen wie Asthma, von Störungen der Darmmotilität wie etwa des irritablen Colons oder der Inkontinenz der Harnblase.

Die Erfindung betrifft weiterhin ein Verfahren zur Herstellung der Verbindung I, dadurch gekennzeichnet, daß man

a) Verbindungen der Formel II

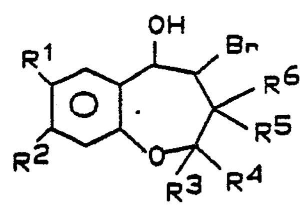

(II),

in denen $R^1$ bis $R^6$ wie oben definiert sind,
umsetzt mit Verbindungen der Formel III oder IV

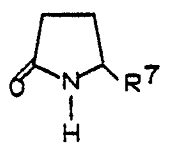

(III)

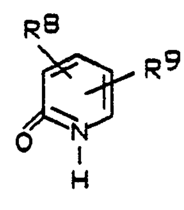

(IV)

oder daß man

b) Verbindungen der Formel V

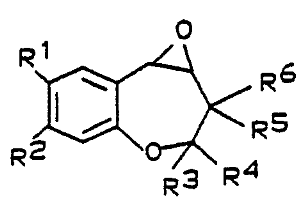

(V),

in denen $R^1$ bis $R^6$ wie oben definiert sind,
umsetzt mit Verbindungen der Formel III oder IV

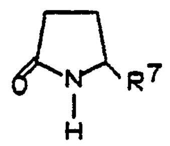 (III)

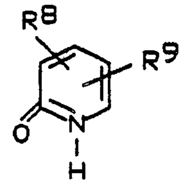 (IV),

oder daß man

c) Verbindungen der Formel II

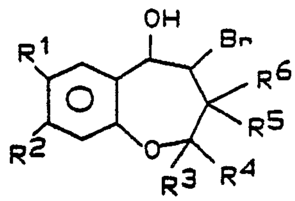 (II),

in denen $R^1$ bis $R^6$ wie oben definiert sind,
umsetzt mit Verbindungen der Formel VI und VII

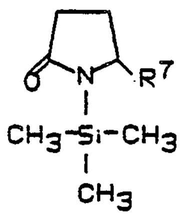 (VI)

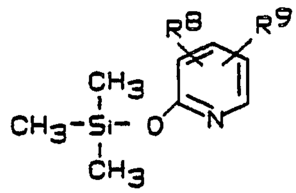 (VII),

oder daß man

d) Verbindungen der Formel V

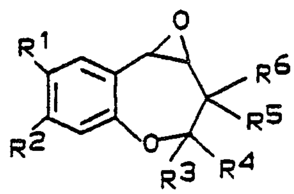 (V),

in denen $R^1$ bis $R^6$ wie oben definiert sind,
umsetzt mit Verbindungen der Formel VI und VII

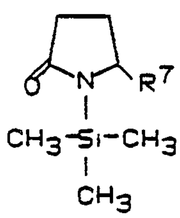 (VI)

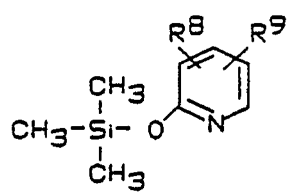 (VII).

Werden die Verbindungen I nach den Methoden a) oder b) hergestellt, so geschieht dies dadurch, daß man die Verbindungen II oder V in einem geeigneten Lösemittel wie etwa Dimethylsulfoxid, Dimethylformamid, Tetrahydrofuran oder N-alkylierten Harnstoffen, beispielsweise 1,3-Dimethyltetrahydro-2(1H)-pyrimidinon (DMPU), umsetzt mit den Verbindungen III oder IV, vorzugsweise unter Einwirkung von starken Basen wie etwa Natriumhydrid, Kaliumhydrid, Lithiumdiisopropylamid, Lithium-bis-(trimethylsilyl)-amid, Kalium-bis-(trimethylsilyl)-amid oder ähnlichen, für Lactam-N-Alkylierungen bekanntermaßen geeigneten Basen. Die

Reaktionstemperatur ist dabei im weiten Bereich variierbar, vorzugsweise wird zwischen 0° C und Zimmertemperatur gearbeitet oder bei Temperaturen, die leicht oberhalb der Zimmertemperatur liegen können.

Verbindungen, die nach den Methoden a) oder b) nur schwierig herstellbar sind, können nach dem Verfahren c) oder d) zugänglich gemacht werden. In diesem Fall rührt man die Verbindungen II oder V zusammen mit den Verbindungen VI oder VII in Gegenwart katalytischer oder molarer Mengen eines Desilylierungsmittels wie Kalium-tert.-butylat oder Tetrabutylammoniumfluoridtrihydrat in einem dipolar aprotischen Lösemittel wie THF und dergleichen. Es ist möglich, die Umsetzung auch ohne Lösemittelzusätze in Gegenwart eines Überschusses der flüssigen Verbindungen VI oder VII durchzuführen. Die Temperatur kann dabei in weiten Grenzen variieren.

So erhält man in vielen Fällen bereits bei Zimmertemperatur die erfindungsgemäßen Verbindungen I, in anderen Fällen erst nach Erhitzen auf 60 - 80° C. In einzelnen Fällen sind sogar noch höhere Temperaturen notwendig.

Eine sehr vorteilhafte Arbeitsweise besteht bei den Umsetzungen nach der Methode d) darin, daß man Verbindungen der Formel V in einem Überschuß der flüssigen Verbindungen VI oder VII suspendiert und katalytische Mengen Tetrabutylammoniumfluorid-trihydrat zusetzt, wobei zunächst die Verbindungen der Formel Ia entstehen,

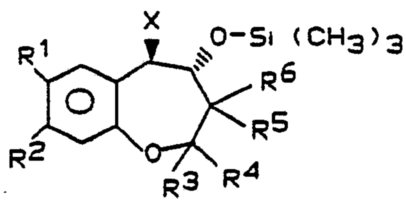

(Ia)

die als gut zu reinigende Zwischenprodukte mit molaren Mengen Tetrabutylammoniumfluorid-trihydrat in THF in die erfindungsgemäßen Verbindungen I überführt werden können.

Verbindungen der Formel III und V sind in vielen Fällen käuflich oder können einfach nach literaturbekannten Methoden synthetisiert werden.

Die Silylverbindungen der Formel VI und VII können in an sich bekannter Weise aus Verbindungen der Formel III und IV hergestellt werden, beispielsweise durch Erhitzen mit 1,1,1,3,3,3-Hexamethyldisilazan und anschließender Destillation.

Die Verbindungen der Formel II und V sind überwiegend neu und können wie nachfolgend beschrieben erhalten werden.

Ausgehend von geeignet substituierten 4-Phenoxybuttersäuren der Formel VIII können die entsprechenden Carbonsäurechloride der Formel IX hergestellt werden, die sich bequem in an sich bekannter Weise in Gegenwart von Aluminiumtrichlorid in 1,2-Dichlorethan zu 2,3-Dihydro-1-benzoxepin-5(4H)-onen der Formel X cyclisieren lassen.

Alternativ gelingt die Cyclisierung in einzelnen Fällen auch durch Erhitzen der Carbonsäuren der Formel VIII in Polyphosphorsäure.

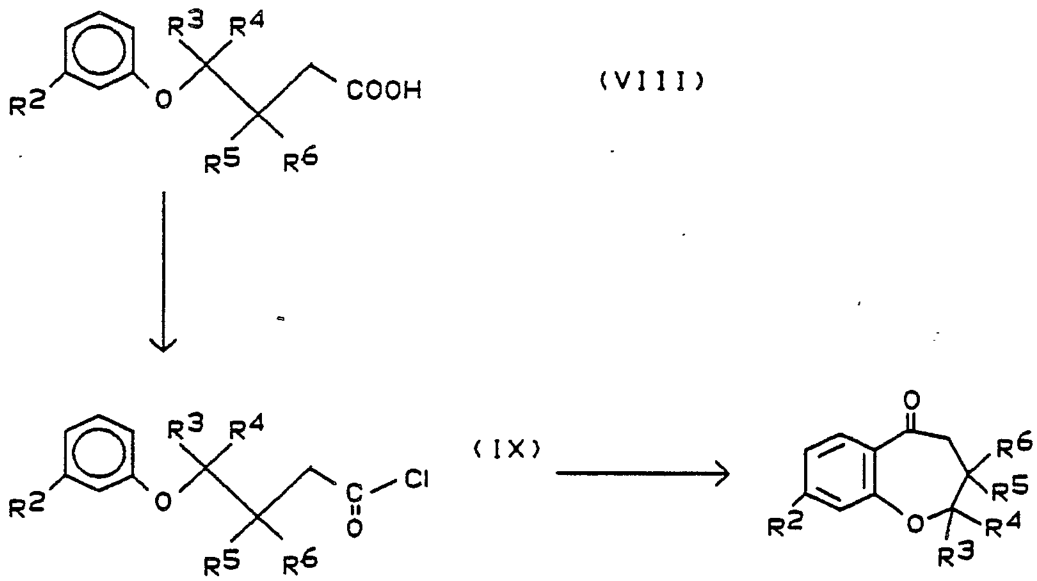

(VIII)

(IX)

(X)

Die Ketone der Formel X können in an sich bekannter Weise in die Alkohole der Formel XI überführt werden. Hierzu eignet sich beispielsweise $NaBH_4$ in Methanol oder Ethanol besonders gut.

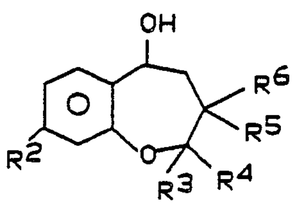

(XI)

Die Einführung der Substituenten R[1] mit den oben definierten Bedeutungen kann von Fall zu Fall entweder an Verbindungen der Formel X oder XI vorgenommen werden. So können beispielsweise die 2,3-Dihydro-1-benzoxepin-5(4H)-one der Formel X in konzentrierter Schwefelsäure durch Zugabe von festem Natriumnitrat bei 0° C bequem zu Verbindungen der Formel XII nitriert werden.

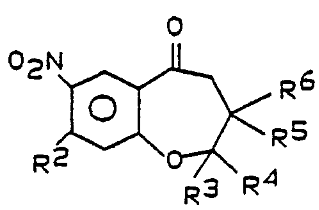

(XII)

Die Einführung eines Bromatoms in die 7-Position des 2,3,4,5-Tetrahydrobenzoxepinsystems gelingt durch Umsetzung der Verbindungen der Formel XI mit N-Bromsuccinimid in Eisessig bei 10 - 20° C. Man erhält auf diese Weise Verbindungen der Formel XIII

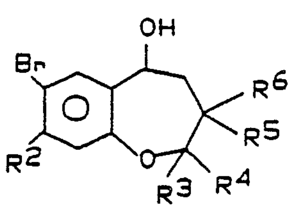

(XIII).

Die Verbindungen der Formel XIII eliminieren bei Zugabe katalytischer Mengen p-Toluolsulfonsäure in Toluol in der Siedehitze Wasser, das sich durch azeotrope Destillation entfernen läßt. Auf diese Weise

lassen sich Verbindungen der Formel XIV herstellen.

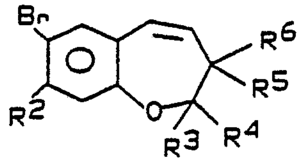

(XIV)

Die Verbindungen der Formel XIV lassen sich bei -78°C in THF mit Hilfe von 2 Äquivalenten tert.-Butyllithium in 7-Stellung metallieren. Auf diese Weise erhält man Aryllithiumverbindungen der Formel XV, die sich in an sich bekannter Weise mit einer Vielzahl von Elektrophilen zu Verbindungen der Formel XVI umsetzen lassen.

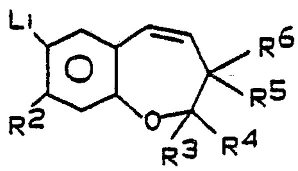

(XV)

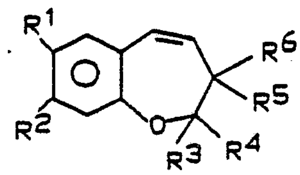

(XVI)

Beispielsweise erhält man durch Umsetzung von Verbindungen der Formel XV mit Diaryldisulfiden die Verbindungen der Formel XVII

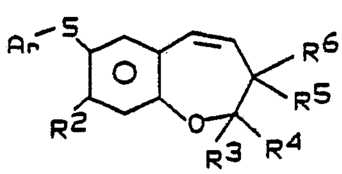

(XVII),

wobei Ar in der oben genannten Bedeutung substituiert sein kann.

Die Verbindungen der Formel XVII können in an sich bekannter Weise selektiv zu Sulfoxiden bzw. Sulfonen der Formel XIX und XVIII umgesetzt werden, z. B. durch Oxidation mit Wasserstoffperoxid in Eisessig.

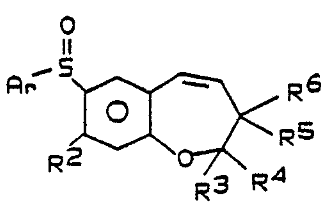

(XIX)

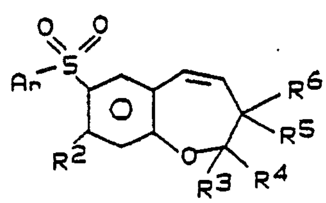

(XVIII)

Die Aryllithiumverbindungen der Formel XV lassen sich u. a. auch mit Alkylhalogeniden, vorzugsweise mit Iodiden, Aldehyden, Amiden, Kohlensäurederivaten, Kohlendioxid, Halogenen umsetzen. Dadurch können die oben definierten Substituenten $R^1$ oft in einem Reaktionsschritt in Verbindungen der Formel XIV eingeführt werden oder es ergeben sich nach Anwendung organischer Standardreaktionen, wie oben beispielshaft gezeigt, die gewünschten Substituenten $R^1$ in Position 7.

Es ist auch möglich, die Einführung zahlreicher Substituenten $R^1$ ausgehend von Verbindungen der Formel XIII vorzunehmen. Dazu metalliert man Verbindungen der Formel XIII bei -90°C in THF mit 3 Äquivalenten tert.-Butyllithium und erhält die Dilithiumverbindungen der Formel XX,

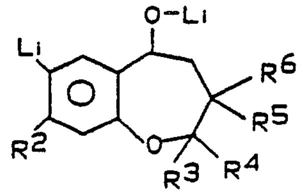

(XX)

die sich, wie oben beispielhaft an der Verbindung XV gezeigt, in an sich bekannter Weise zu Verbindungen der Formel XXI umsetzen lassen.

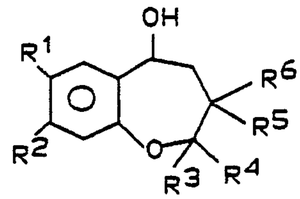

(XXI)

Die Wassereliminierung an Verbindungen der Formel XXI zu Verbindungen der Formel XVI erfolgt wie oben beschrieben.

Die Verbindungen der Formel XVI können in an sich bekannter Weise mit N-Bromsuccinimid in wäßrigem Dimethylsulfoxid zu Verbindungen der Formel II umgesetzt werden.

Epoxide der Formel V sind in an sich bekannter Weise aus Bromhydrinen der Formel II zugänglich, beispielsweise durch Einwirkung von Basen auf Verbindungen der Formel II.

Enantiomerenreine Endprodukte I können aus racemischen Endprodukten I durch gängige Racematspaltungsmethoden, wie etwa die chromatographische Trennung unter Verwendung von chiralen Phasen oder die Derivatisierung der racemischen Produkte mit optisch einheitlichen Säurederivaten oder mit optisch einheitlichen Isocyanaten erhalten werden. Die dabei erhaltenen diastereoisomeren Isocyanate oder Ester lassen sich durch gängige Methoden (Kristallisation oder Chromatographie) trennen und unter Abspaltung der optisch aktiven Hilfsgruppe an der 4-OH-Funktion in die optisch einheitlichen Endverbindungen I umwandeln. Als besonders vorteilhaft hat sich hierbei die Trennung der diastereomeren 4-Menthoxyacetate erwiesen.

Die erfindungsgemäßen Verbindungen I können wie bereits erwähnt als Antihypertensiva, als Coronartherapeutika, als Mittel zur Behandlung der Herzinsuffizienz, der cerebralen bzw. peripheren Durchblutungsstörungen oder von Darmmotilitätsstörungen, vorzeitiger Wehentätigkeit, Obstruktionen der Atemwege oder der ableitenden Harnwege oder der Gallenwege oder als Spasmolytika eingesetzt werden.

Arzneimittel, die die Verbindung I enthalten, können dabei oral, parenteral, intravenös, rektal oder durch Inhalation appliziert werden, wobei die bevorzugt Applikationsform von der zu behandelnden Krankheit abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann aufgrund seines Fachwissens geläufig. Neben Lösemitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen wie Trägerstoffen, Stabilisatoren oder inerten Verdünnungsmitteln vermischt und durch die üblichen Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage Wasser, physiologische Kochsalzlösung oder Alkohole, z. B. Ethanol, Propanol, Glycerin, daneben auch Zuckerlösungen wie

Glucose- oder Mannitlösungen, oder auch eine Mischung aus den verschiedenen genannten Lösungsmitteln.

Als pharmazeutische Formulierungen für die Verabreichung in Form von Aerosolen oder Sprays sind geeignet z. B. Lösungen, Suspensionen oder Emulsionen des Wirkstoffs der Formel I in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelden Krankheit sowie von Geschlecht, Alter, Gewicht und individueller Ansprechbarkeit des zu behandelndes Säugers. Im Durchschnitt beträgt die empfohlene tägliche Dosis einer Verbindung der Formel I bei einem etwa 75 kg schweren Patienten mindestens 0,1 mg, vorzugsweise mindestens 1 mg, bis maximal 100 mg, vorzugsweise bis maximal 10 mg. Bei akuten Ausbrüchen der Krankheit, etwa bei Asthmaanfällen oder bei Nierenkoliken, können dabei mehrere, z. B. bis zu 4 Einzeldosen pro Tag, erforderlich sein, während zur Prophylaxe auch eine Dosierung ausreichen kann.

Erfindungsgemäß können beispielsweise die in der folgenden Tabelle zusammengestellten Verbindungen der Formel I erhalten werden.

7-Nitro-trans-5-(2-oxo-pyrrolidin-1-yl)-2,3,4,5-tetrahydro-1-benzoxepin-4-ol
Trans-5-(2-oxo-pyrrolidin-1-yl)-7-phenylsulfinyl-2,3,4,5-tetrahydro-1-benzoxepin-4-ol
Trans-5-(2-oxo-pyrrolidin-1-yl)-7-phenylsulfonyl-2,3,4,5-tetrahydro-1-benzoxepin-4-ol
Trans-5-(2-oxo-pyrrolidin-1-yl)-7-phenylcarbonyl-2,3,4,5-tetrahydro-1-benzoxepin-4-ol
7-(2-Fluor-phenylcarbonyl)-trans-5-(2-oxo-pyrrolidin-1-yl)-2,3,4,5-tetrahydro-1-benzoxepin-4-ol
7-Cyan-trans-5-(2-oxo-pyrrolidin-1-yl)-2,3,4,5-tetrahydro-1-benzoxepin-4-ol
7-Cyan-trans-5-(1,2-dihydro-2-oxo-pyrid-1-yl)-2,3,4,5-tetrahydro-1-benzoxepin-4-ol
Trans-5-(5-chlor-1,2-dihydro-2-oxo-pyrid-1-yl)-7-cyan-2,3,4,5-tetrahydro-1-benzoxepin-4-ol
7-Cyan-trans-5-(5-nitro-1,2-dihydro-2-oxo-pyrid-1-yl)-2,3,4,5-tetrahydro-1-benzoxepin-4-ol
Trans-5-(5-nitro-1,2-dihydro-2-oxo-pyrid-1-yl)-7-phenylsulfinyl-2,3,4,5-tetrahydro-1-benzoxepin-4-ol
Trans-5-(5-chlor-1,2-dihydro-2-oxo-pyrid-1-yl)-7-phenylsulfinyl-2,3,4,5-tetrahydro-1-benzoxepin-4-ol
Trans-5-(5-nitro-1,2-dihydro-2-oxo-pyrid-1-yl)-7-phenylsulfonyl-2,3,4,5-tetrahydro-1-benzoxepin-4-ol
Trans-5-(5-chlor-1,2-dihydro-2-oxo-pyrid-1-yl)-7-phenylsulfonyl-2,3,4,5-tetrahydro-1-benzoxepin-4-ol
Trans-5-(1,2-dihydro-2-oxo-pyrid-1-yl)-7-(4-chlorphenylcarbonyl)-2,3,4,5-tetrahydro-1-benzoxepin-4-ol

Sind in den nachfolgend aufgeführten Beispielen Asymmetriezentren vorhanden, so sollen die jeweiligen Formelbilder nur relative Konfigurationen zum Ausdruck bringen.

Die Angabe der chemischen Verschiebung erfolgt in ppm gegenüber TMS als internem Standard.

**Beispiel 1**

Trans-5-(2-oxo-pyrrolidin-1-yl)-2,3,4,5-tetrahydro-1-benzoxepin-4-ol

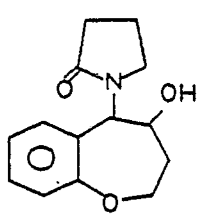

3,24 g (20 mmol) 4,5-Epoxy-2,3,4,5-tetrahydro-1-benzoxepin und 9,60 g (61 mmol) N-Trimethylsilyl-2-oxo-pyrrolidin werden in 8 ml trockenem THF gelöst und unter Kühlung 6,31 g (20 mmol) Tetrabutylammoniumfluorid-Trihydrat auf einmal zugesetzt und mehrere Stunden auf 100 °C erhitzt (DC-Kontrolle). Der Ansatz wird mit Eiswasser hydrolysiert und mehrmals mit Diethylether extrahiert. Die vereinigten Diethyletherextrakte werden mit gesättigter Natriumchloridlösung gewaschen und über Natriumsulfat getrocknet. Nach Filtration und Abdampfen des Lösungsmittels im Vakuum wird der Rückstand

in Diisopropylether/Essigester kristallisiert. Man erhält Kristalle vom Smp. 151 - 153 °C.

| $C_{14}H_{17}NO_3$ (247.298): | | | |
|---|---|---|---|
| Ber. | C 68,00 | H 6,93 | N 5,66 |
| Gef. | C 67,9 | H 7,0 | N 5,6 |

**Beispiel 2**

7-Chlor-trans-5-(2-oxo-pyrrolidin-1-yl)-2,3,4,5-tetrahydro-1-benzoxepin-4-ol

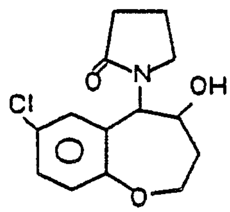

Zu 1,40 g (5 mmol) Trans-5-brom-7-chlor-4-hydroxy-2,3,4,5-tetrahydro-1-benzoxepin werden unter Argon bei 0 °C 5 ml (5 mmol) Bis-(trimethylsilyl)-lithium-amid (1 M Lösung in THF) unter Rühren zugetropft. Nach 2 stündigem Rühren setzt man 0,43 g (5 mmol) 2-Pyrrolidinon und anschließend nochmals 5 ml Bis-(trimethylsilyl)-lithium-amid zu und erhitzt für mehrere Stunden auf 110 °C (DC-Kontrolle). Man gibt den Ansatz auf Eiswasser und extrahiert mehrmals mit Essigester. Die vereinigten Essigesterextrakte werden mit Wasser und gesättigter Natriumchloridlösung gewaschen, über Natriumsulfat getrocknet, filtriert und im Vakuum eingedampft. Der Rückstand wird mit heißem Methanol/Essigester verrieben und abgesaugt. Man erhält Kristalle vom Smp. 186 ° - 188 °C.

| $C_{14}H_{16}ClNO_3$ (281.747): | | | |
|---|---|---|---|
| Ber. | C 59.68 | H 5,72 | N 4,97 |
| Gef. | C 59,4 | H 5,7 | N 4,9 |

**Beispiel 3**

Trans-5-(1,2-dihydro-2-oxo-pyrid-1-yl)-2,3,4,5-tetrahydro-1-benzoxepin-4-ol

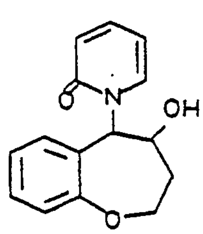

Zu einer Mischung aus 3,24 g (20 mmol) 4,5-Epoxy-2,3,4,5-tetrahydro-1-benzoxepin und 10 g (60

mmol) 2-Trimethylsilyloxy-pyridin werden 6,40 g (20 mmol) Tetrabutylammoniumfluorid-Trihydrat zugesetzt und unter Rühren auf 80 - 90 °C erhitzt. Nach 5 Stunden Reaktionszeit rührt man den Ansatz langsam in Eiswasser und saugt den ausgefallenen Feststoff ab, den man aus Methanol unter Zusatz von geringen Mengen Dioxan umkristallisiert. Man erhält Kristalle vom Schmelzpunkt 232 - 234 °C.

| $C_{15}H_{15}NO_3$ (257.29): | | | |
|---|---|---|---|
| Ber. | C 70,02 | H 5,88 | N 5,44 |
| Gef. | C 70,1 | H 5,9 | N 5,4 |

$^1$H-NMR (60 MHz/d$^6$-DMSO): $\delta$ = 7,67 - 6,63 (m,6H); $\delta$ = 6,53 - 5,93 (m,3H); $\delta$ = 5,23 (d,J = 5Hz, 1H); $\delta$ = 4,50 - 3,63 (m,3H); $\delta$ = 2,27 - 1,77 (m,2H).

**Beispiel 4**

Trans-5-(5-chlor-1,2-dihydro-2-oxo-pyrid-1-yl)-2,3,4,5-tetrahydro-1-benzoxepin-4-ol

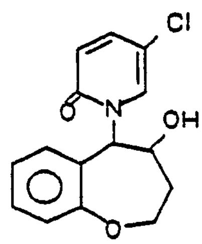

Zu einer Mischung aus 10,54 g (65 mmol) 4,5-Epoxy-2,3,4,5-tetrahydro-1-benzoxepin und 19,4 g(96 mmol) 5-Chlor-2-trimethylsilyloxy-pyridin werden 22,7 g (72 mmol) Tetrabutylammoniumfluorid-Trihydrat zugesetzt und 3 Stunden auf 90 °C erhitzt. Den Ansatz rührt man in Wasser ein und extrahiert mehrmals mit Essigester. Man wäscht die vereinigten organischen Phasen mit Wasser, gesättigter Natriumchloridlösung und dampft das Lösungsmittel im Vakuum ab. Den Rückstand kristallisiert man in Essigsäure-n-butylester. Man erhält Kristalle vom Schmelzpunkt 175,5 - 177,5 °C.

| $C_{15}H_{14}ClNO_3$ (291.74): | | | |
|---|---|---|---|
| Ber. | C 61,75 | H 4,84 | N 4,80 |
| Gef. | C 61,7 | H 4,9 | N 4,8 |

$^1$H-NMR (270 MHz/d$^6$-DMSO) : $\delta$ = 7,83 (d, J = 3 Hz, 1H), $\delta$ = 7,53 (d,d, $J_1$ = 10 Hz, $J_2$ = 3Hz, 1H); $\delta$ = 7,28 (t,d, $J_1$ = 8 Hz, $J_2$ = 1 Hz, 1H); $\delta$ = 7,07 (t, J = 8 Hz, 1H); $\delta$ = 7,06 (t,d, $J_1$ = 8 Hz, $J_2$ = 1 Hz, 1H); $\delta$ = 6,75 (d, J = 8 Hz, 1H); $\delta$ = 6,52 (d, J = 10 Hz, 1H); $\delta$ = 5,98 (d, J = 5 Hz, 1H); $\delta$ = 5,35 (d, J = 5 Hz, 1H); $\delta$ = 4,25 (m, 2H); $\delta$ = 3,88 (d,d,d, $J_1$ = 10, $J_2$ = $J_3$ = 3 Hz, 1 H); $\delta$ = 2,18 - 1,92 (m, 2H).

**Beispiel 5**

Trans-5-(1,2-dihydro-5-nitro-2-oxo-pyrid-1-yl)-2,3,4,5-tetrahydro-1-benzoxepin-4-ol

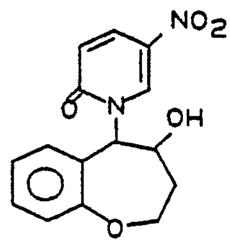

Zu 1,61 g (56 mmol) Natriumhydrid (80 %ige Dispersion in Mineralöl) in 30 ml DMPU (1,3-Dimethyl-tetrahydro-2(1H)-pyrimidin-on) gibt man unter Argon portionsweise 7,85 g (56 mmol) 2-Hydroxy-5-nitro-pyridin. Nach dem Abklingen der Gasentwicklung wird noch 20 Minuten nachgerührt. Dann tropft man bei Zimmertemperatur eine Lösung des 4,5-Epoxy-2,3,4,5-tetrahydro-1-benzoxepins in 10 ml DMPU zu. Anschließend wird 6 Stunden auf 95° C erhitzt. Der Ansatz wird unter kräftigem Rühren in Eis/Wasser gegossen und vom ausgefallenen Feststoff abgesaugt. Der Feststoff wird über Kieselgel chromatographiert (Essigester/Cyclohexan 2 : 1) und das so erhältliche hellgelbe Öl in Methyl-tert.-butylether kristallisiert. Smp. 152 - 155° C.

| $C_{15}H_{14}N_2O_5$ (302.29): | | | |
|---|---|---|---|
| Ber.: | C 59,60 | H 4,67 | N 9,27 |
| Gef.: | C 59,5 | H 4,6 | N 9,0 |

$^1$H-NMR (270 MHz/d$^6$-DMSO): $\delta$ = 8,93 (d, J = 3 Hz, 1H); $\delta$ = 8,19 (d,d, J = 10 Hz, $J_2$ = 3 Hz, 1H); $\delta$ = 7,32 (t,d, $J_1$ = 8 Hz, $J_2$ = 1 Hz, 1H); $\delta$ = 7,11 (d, J = 8 Hz, 1H); $\delta$ = 7,07 (t,d, $J_1$ = 8 Hz, $J_2$ = 1 Hz, 1H); $\delta$ = 6,89 (d, J = 8 Hz, 1H); $\delta$ = 6,61 (d, J = 10 Hz, 1H); $\delta$ = 6,09 (d, J = 8 Hz, 1H); $\delta$ = 5,51 (d, J = 6 Hz, 1H); $\delta$ = 4,31 (m, 2H); $\delta$ = 3,95 (d,d,d, $J_1$ = 8 Hz, $J_2$ = $J_3$ = 3 Hz, 1H); $\delta$ = 2,16 (m, 1H), $\delta$ = 1,99 (m, 1H).

**Beispiel 6**

7-Nitro-trans-5-(5-chlor-1,2-dihydro-2-oxo-pyrid-1-yl)-2,3,4,5-tetrahydro-1-benzoxepin-4-ol

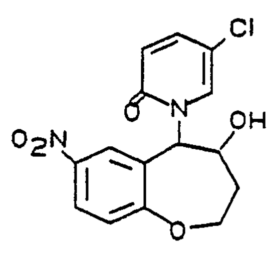

Zu einer Mischung von 3,93 g (19 mmol) 4,5-Epoxy-7-nitro-2,3,4,5-tetrahydro-1-benzoxepin und 8,06 g (40 mmol) 5-Chlor-2-trimethylsilyl-pyridin gibt man unter Rühren 6,63 g (21 mmol) Tetrabutylammoniumfluorid-Trihydrat zu und erhitzt 5 Stunden auf 60° C. Den Ansatz rührt man dann in Eiswasser ein und extrahiert mehrmals mit Essigester. Die vereinigten Essigesterextrakte werden mit Wasser und gesättigter Natriumchloridlösung gewaschen, über Natriumsulfat getrocknet, filtriert und im Vakuum eingedampft. Das zurückbleibende zähe Öl kristallisiert nach einigem Stehen. Man verreibt mit Essigester und saugt ab. Man erhält Kristalle vom Schmelzpunkt 219 - 222° C.

| $C_{15}H_{13}ClN_2O_5$ (336.74) | | | |
|---|---|---|---|
| Ber.: | C 53,50 | H 3,89 | N 8,32 |
| Gef.: | C 53,3 | H 3,9 | N 8,2 |

$^1$H-NMR (270 MHz/d$^6$-DMSO): $\delta$ = 8, 16 (d,d, $J_1$ = 8 Hz, $J_2$ = 3 Hz, 1H); $\delta$ = 7,99 (d, J = 3 Hz, 1H); $\delta$ = 7,60 (d,d, $J_1$ = 10 Hz, $J_2$ = 3 Hz, 1H); $\delta$ = 7,51 (d, J = 3 Hz, 1H); $\delta$ = 7,27 (d, J = 8 Hz, 1H); $\delta$ = 6,57 (d, J = 10 Hz, 1H); $\delta$ = 6,00 (d, J = 9 Hz, 1H); $\delta$ = 5,49 (d, J = 6 Hz, 1H); $\delta$ = 4,51 (m, 1H); $\delta$ = 4,40 (m, 1H); $\delta$ = 4,04 (m, 1H); $\delta$ = 2,21 (m, 1H); $\delta$ = 2,04 (m, 1H).

**Beispiel 7**

7-Nitro-trans-5-(1,2-dihydro-2-oxo-pyrid-1-yl)-2,3,4,5-tetrahydro-1-benzoxepin

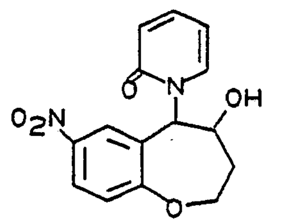

Man erhält die Verbindung analog Beispiel 6.
Smp. 200 - 203° C.

| $C_{15}H_{14}N_2O_5$ (302.29) | | | |
|---|---|---|---|
| Ber.: | C 59,60 | H 4,67 | N 9,27 |
| Gef.: | C 59,5 | H 4,9 | N 9,0 |

$^1$H-NMR (270 MHz/d$^6$-DMSO): $\delta$ = 8,14 (d,d, $J_1$ = 8 Hz, $J_2$ = 3 Hz, 1H); $\delta$ = 7,74 (d,d, $J_1$ = 7 Hz $J_2$ = 1 Hz, 1H); $\delta$ = 7,56 - 7,49 (m, 2H); $\delta$ = 7,25 (d, J = 8 Hz, 1H); $\delta$ = 6,5 (d, J = 10 Hz, 1H); $\delta$ = 6,37 (t,d, $J_1$ = 7 Hz, $J_2$ = 1 Hz, 1H); $\delta$ = 6,10 (d, J = 8 Hz, 1H); $\delta$ = 5,54 (brS, 1H); $\delta$ = 4,53 (m, 1H); $\delta$ = 4,41 (m, 1H); $\delta$ = 4,07 (m, 1H); $\delta$ = 2,21 (m, 1H); $\delta$ = 2,05 (m, 1H).

**Beispiel 8**

Trans-5-(1,2-dihydro-2-oxo-pyrid-1-yl)-7-phenylsulfinyl-2,3,4,5-tetrahydro-1-benzoxepin-4-ol

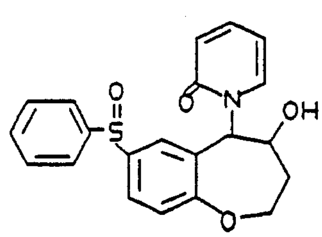

Zu einer Mischung von 4,00 g (10,9 mmol) Trans-4-brom-5-hydroxy-7-phenylsulfinyl-2,3,4,5-tetrahydro-1-benzoxepin und 6,40 g (38,3 mmol) 2-Trimethylsilyloxypyridin werden 8,60 g (27,3 mmol)

Tetrabutylammoniumfluorid-Trihydrat zugegeben und anschließend 5 Stunden bei 60 °C gerührt. Die erkaltete sirupöse Flüssigkeit wird auf Eiswasser gegossen und mehrmals mit Essigester extrahiert. Die vereinigten Essigesterextrakte werden mit Wasser und gesättigter Natriumchloridlösung gewaschen, über Natriumsulfat getrocknet, filtriert und eingedampft. Der schaumige Rückstand wird in Butylacetat kristallisiert. Man erhält weiße Kristalle vom Smp. 181 - 184 °C.

| $C_{21}H_{19}NO_4S$ (381.45) | | | |
|---|---|---|---|
| Ber.: | C 66,1 | H 5,0 | N 3,6 |
| Gef.: | C 65,5 | H 5,1 | N 3,5 |

$^1$H-NMR (270 MHz/d$^6$-DMSO): $\delta$ = 7,62 - 7,43 (m, 8H); $\delta$ = 7,18 (d,d, $J_1$ = 8 Hz, $J_2$ = 3 Hz, 1H); $\delta$ = 7,01 (d, J = 2 Hz, 1H); $\delta$ = 6,45 (m, 1H); 6,28 (m, 1H); 6,05 (d, J = 8 Hz, 1H); $\delta$ = 5,33 (d,d, $J_1$ = 6 Hz, $J_2$ = 2 Hz, 1H); $\delta$ = 4,34 (m, 1H); $\delta$ = 4,21 (m, 1H); $\delta$ = 3,94 (m, 1H); $\delta$ = 2,18 - 2,06 (m, 1H); $\delta$ = 2,04 - 1,90 (m, 1H).

**Beispiel 9**

Trans-5-(1,2-dihydro-2-oxo-pyrid-1-yl)-7-phenylsulfonyl-2,3,4,5-tetrahydro-1-benzoxepin-4-ol

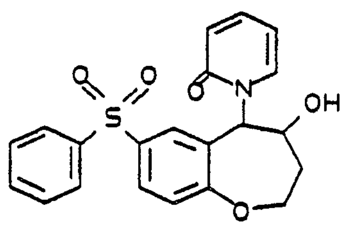

erhält man analog Beispiel 8 aus Trans-4-brom-5-hydroxy-7-phenylsulfonyl-2,3,4,5-tetrahydro-1-benzoxepin. Smp. 190 - 193 °C (nach Ausrühren in Essigester).

| $C_{21}H_{19}NO_5S$ (397.46) | | | |
|---|---|---|---|
| Ber.: | C 63,4 | H 4,81 | N 3,52 |
| Gef.: | C 63,1 | H 4,9 | N 3,6 |

$^1$H-NMR (270 MHz/d$^6$-DMSO) : $\delta$ = 7,86 - 7,77 (m, 3H); $\delta$ = 7,72 - 7,46 (m, 5H); $\delta$ = 7,22 (d, J = 8 Hz, 1H); $\delta$ = 7,19 (d, J = 2 Hz, 1H); $\delta$ = 6,48 (d, J = 10 Hz, 1H); $\delta$ = 6,34 (t, d, $J_1$ = 6 Hz, J2 = 1 Hz, 1H); $\delta$ = 6,04 (d, J = 8 Hz, 1H); $\delta$ = 5,36 (d, J = 6 Hz, 1H); $\delta$ = 4,41 (m, 1H); $\delta$ = 4,27 (m, 1H); $\delta$ = 3,99 (m, 1H); $\delta$ = 2,27 - 2,10 (m, 1H); $\delta$ = 2,07 - 1,92 (m, 1H).

**Beispiel 10**

Trans-5-(1,2-dihydro-2-oxo-pyrid-1-yl)-7-(2-fluorbenzoyl)-2,3,4,5-tetrahydro-1-benzoxepin-4-ol

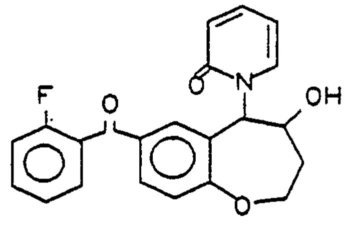

erhält man analog Beispiel 8 aus Trans-4-brom-5-hydroxy-7-(2-fluorbenzoyl)-2,3,4,5-tetrahydro-1-benzoxepin. Smp. 219 - 221 °C (Methanol).

| $C_{22}H_{18}F_1O_4$ (379,39) | | | |
|---|---|---|---|
| Ber.: | C 69,6 | H 4,78 | N 3,69 |
| Gef.: | C 69,3 | H 4,9 | N 3,7 |

$^1$H-NMR (270 MHz/d$^6$-DMSO) : δ = 7,69 - 7,56 (m, 3H); δ = 7,47 - 7,27 (m, 4H); δ = 7,18 (d, J = 8 Hz, 1H); δ = 7,12 (sbr, 1H); δ = 6,42 (d, J = 10 Hz, 1H); δ = 6,24 (t, d, $J_1$ = 7 Hz, $J_2$ = 2 Hz, 1H); δ = 6,10 (d, J = 8 Hz, 1H); δ = 5,35 (d, J = 6 Hz, 1H); δ = 4,47 (m, 1H); δ = 4,27 (m, 1H); δ = 4,01 (m, 1H); δ = 2,21 (m, 1H); δ = 2,06 (m, 1H).

**Beispiel 11**

Trans-5-(1,2-dihydro-2-oxo-pyrid-1-yl)-7-(2-trifluormethylbenzoyl)-2,3,4,5-tetrahydro-1-benzoxepin-4-ol

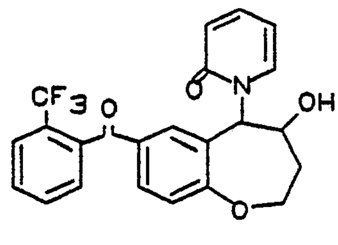

erhält man analog Beispiel 8 aus Trans-4-brom-5-hydroxy-7-(2-trifluormethyl-benzoyl)-2,3,4,5-tetrahydro-1-benzoxepin. Smp. 176 - 179 °C (Methyl-tert. butylether).

| $C_{23}H_{18}F_3N_1O_4$ (429,41) | | | |
|---|---|---|---|
| Ber.: | C 64,33 | H 4,2 | N 3,2 |
| Gef.: | C 64,6 | H 4,1 | N 3,1 |

$^1$H-NMR (270 MHz/$CDCl_3$): δ = 7,74 (m, 2H); δ = 7,63 - 7,49 (m, 3H); δ = 7,37 - 7,24 (m, 3H); δ = 7,16 (d, J = 8 Hz, 1H); δ = 6,55 (d, J = 10 Hz, 1H); δ = 6,15 (m,2H); δ = 4,45 (m,1H); δ = 4,38 - 4,15 (m, 3H); δ = 2,27 - 2,01 (m, 2H).

**Beispiel 12**

Trans-7-nitro-5-(2-oxo-pyrrolidin-1-yl)-2,3,4,5-tetrahydro-1-benzoxepin-4-ol

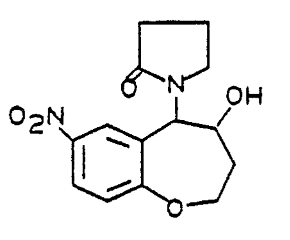

Man suspendiert 6,22 g (30 mmol) 4,5-Epoxy-7-nitro-2,3,4,5-tetrahydro-1-benzoxepin in 28,3 g (180

mmol) N-Trimethylsilyl-2-oxo-pyrrolidin und gibt 0,95 g (3 mmol) Tetrabutylammoniumfluorid-trihydrat zu. Nach kurzer Zeit wird der Ansatz dunkelbraun und homogen. Man rührt bis zur vollständigen Umsetzung bei Zimmertemperatur (DC-Kontrolle). Man hydrolysiert, extrahiert mehrmals mit Essigester und wäscht die vereinigten Essigesterextrakte mit Wasser und gesättigter Natriumchloridlösung. Nach Trocknen über Natriumsulfat und Abdampfen des Lösungsmittels im Vakuum verrührt man den Rückstand mit Diethylether. Die ausgefallenen Kristalle werden abgesaugt und mit wenig kaltem Diethylether nachgewaschen.
Ausbeute: 7,30 g Trans-7-nitro-5-(2-oxo-pyrrolidin-1-yl)-4-trimethylsilyloxy-2,3,4,5-tetrahydro-1-benzoxepin vom Smp. 159 - 161 °C.

| $C_{17}H_{24}N_2O_5Si$ (364,48) | | | |
|---|---|---|---|
| Ber.: | C 56,02 | H 6,63 | N 7,68 |
| Gef.: | C 56,1 | H 6,6 | N 7,8 |

$^1$H-NMR (270 MHz/d$^6$-DMSO): δ [ppm] gegenüber Trimethylsilylgruppe
δ = 8,04 (d,d, $J_1$ = 8 Hz, $J_2$ = 3 Hz, 1H); δ = 7,58 (d, J = 3 Hz, 1H); δ = 7,18 (d, J = 8 Hz, 1H); δ = 5,29 (s, 1H); δ = 4,35 (d, br, J = 4 Hz, 1H); δ = 4,30 (d, t, $J_1$ = 12 Hz, $J_2$ = 4 Hz, 1H); δ = 3,81 (t, d, $J_1$ = 12 Hz, $J_2$ = 2 Hz, 1H); δ = 3,55 (m, 2H); δ = 2,41 - 1,90 (m, 6H); δ = 0 (s, 9H).

5,09 g (14 mmol) der obigen Silylverbindung werden zur Abspaltung der Silylgruppe in 15 ml absolutem THF gelöst und 4,42 g (14 mmol) Tetrabutylammoniumfluorid-trihydrat zugesetzt. Man rührt 1 Stunde, hydrolysiert mit verdünnter Ammoniumchloridlösung und extrahiert mehrmals mit Essigester. Die vereinigten Essigesterextrakte werden mit Wasser und gesättigter Natriumchloridlösung gewaschen, über Natriumsulfat getrocknet und das Lösungsmittel wird im Vakuum abgedampft. Der Rückstand wird mit Diisopropylether verrieben und die Kristalle abgesaugt.
Ausbeute 3,65 g.
Smp. 179 - 181 °C.

| $C_{14}H_{16}N_2O_5$ (292.29) | | | |
|---|---|---|---|
| Ber.: | C 57,53 | H 5,5 | N 9,58 |
| Gef.: | C 57,2 | H 5,5 | N 9,6 |

$^1$H-NMR (270 MHz/d$^6$-DMSO): δ = 8,08 (d,d, $J_1$ = 8 Hz, $J_2$ = 3 Hz, 1H); δ = 7,63 (d, J = 3 Hz, 1H); δ = 7,21 (d, J = 8 Hz, 1H); δ = 5,25 (s, 1H); δ = 5,20 (d, J = 4 Hz, 1H); δ = 4,33 (d, t, $J_1$ = 12 Hz, $J_2$ = 4 Hz, 1H); δ = 4,23 (sbr, 1H); δ = 3,93 (t, d, $J_1$ = 12 Hz, $J_2$ = 2 Hz, 1H); δ = 3,69 (t, J = 7 Hz, 2H); δ = 2,38 (m, 2H); δ = 2,31 - 2,03 (m, 3H); δ = 1,91 (m, 1H).

**Beispiel 13**

Trans-7-cyan-5-(2-oxo-pyrrolidin-1-yl)-2,3,4,5-tetrahydro-1-benzoxepin-4-ol

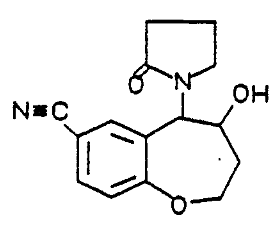

wird in analoger Weise wie unter Beispiel 12 beschrieben aus 7-Cyan-4,5-epoxy-2,3,4,5-tetrahydro-1-benzoxepin hergestellt.
Smp. 168 - 170 °C (Isopropanol).

| $C_{15}H_{16}N_2O_3$ (272,31) | | | |
|---|---|---|---|
| Ber.: | C 66,16 | H 5,92 | N 10,29 |
| Gef.: | C 65,8 | H 5,9 | N 10,0 |

$^1$H-NMR (270 MHz/d$^6$-DMSO): δ = 7,70 (d,d, $J_1$ = 8 Hz, $J_2$ = 3 Hz, 1H); δ = 7,45 (d, J = 3 Hz, 1H); δ = 7,12 (d, J = 8 Hz, 1H); δ = 5,24 (d, J = 5 Hz, 1H); δ = 5,11 (d, J = 8 Hz, 1H); δ = 4,33 (t, d, $J_1$ = 12 Hz, $J_2$ = 2 Hz, 1H); δ = 4,03 (m, 2H); δ = 3,59 (m, 1H); δ = 3,23 (m, 1H); δ = 2,31 (t, J = 8 Hz, 2H); δ = 2,14 (m, 1H); δ = 2,06 - 1,81 (m, 3H).

**Beispiel 14**

Trans-7-nitro-5-(5-(R,S)-methyl-2-oxo-pyrrolidin-1-yl)-2,3,4,5-tetrahydro-1-benzoxepin-4-ol

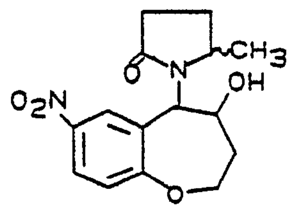

wird analog Beispiel 12 aus 4,5-Epoxy-7-nitro-2,3,4,5-tetrahydro-1-benzoxepin hergestellt.

Man isoliert ausgehend von 10,4 g (50 mmol) 4,5-Epoxy-7-nitro-2,3,4,5-tetrahydro-1-benzoxepin 6,7 g Trans-7-nitro-5-(5-(R,S)-methyl-2-oxo-pyrrolidin-1-yl)-4-trimethylsilyloxy-2,3,4,5-tetrahydro-1-benzoxepin vom Schmelzbereich 179 - 185 °C. Nach Abspaltung der Silylgruppe erhält man nach Kristallisation und Chromatographie an Kieselgel (Essigester/Cyclohexan 7 : 3) die Diastereomeren A und B.

Diasteromer B: Smp. 198 - 200 °C.

$^1$H-NMR (270 MHz/d$^6$-DMSO): δ = 8,11 (d,d, $J_1$ = 8 Hz, $J_2$ = 3 Hz, 1H); δ = 7,76 (d, J = 3 Hz, 1H); δ = 7,24 (d, J = 8 Hz, 1H); δ = 5,65 (sbr, 1H); δ = 4,98 (sbr, 1H); δ = 4,38 - 4,29 (m, 2H); δ = 4,02 (m, 1H); δ = 3,87 (t, d, $J_1$ = 12 Hz, $J_2$ = 2 Hz, 1H); δ = 2,72 - 2,59 (m, 1H); δ = 2,52 - 2,25 (m, 3H); δ = 1,89 (m, 1H); δ = 1,75 (m, 1H); δ = 1,02 (d, J = 7 Hz, 3H).

Diasteromer A: Smp. 145 - 147 °C.

$^1$H-NMR (270 MHz/d$^6$-DMSO): δ = 8,11 (d,d, $J_1$ = 8 Hz, $J_2$ = 3 Hz, 1H); δ = 7,73 (d, J = 3 Hz, 1H); δ = 7,24 (d, J = 8 Hz, 1H); δ = 5,76 (sbr, 1H); δ = 4,91 (sbr, 1H); δ = 4,34 (d, t, $J_1$ = 12 Hz, $J_2$ = 3,5 Hz, 1H); δ = 4,27 (m, 1H); δ = 3,92 (t, d, $J_1$ = 12 Hz, $J_2$ = 2 Hz, 1H); δ = 3,78 (m, 1H); δ = 2,59 (m, 1H); δ = 2,52 - 2,24 (m, 3H); δ = 1,92 (dbr, J = 14 Hz, 1H); δ = 1,75 (m, 1H); δ = 1,28 (d, J = 7 Hz, 3H).

**Darstellung von Vorstufen**

**4-Phenoxybuttersäurechlorid**

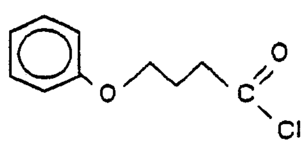

Unter Feuchtigkeitsausschluß werden 252,3 g (1,40 Mol) 4-Phenoxybuttersäure, 154 ml (2,1 Mol)

Thionylchlorid und einige Tropfen Dimethylformamid unter Rückfluß erhitzt. Nach Abklingen der Gasentwicklung (ca. 2 Stunden) wird im Wasserstrahlvakuum fraktioniert. Das Säurechlorid geht bei 149 - 151 °C über.
Ausbeute: 238 g ≙ 1,2 Mol (86 % d. Th.).

**2,3-Dihydro-1-benzoxepin-5(4H)-on**

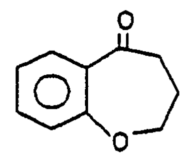

Zu einer auf 0 °C gekühlten Suspension von 192 g Aluminiumtrichlorid in 1 200 ml 1,2-Dichlorethan werden innerhalb von 4,5 Stunden unter Durchleiten von Stickstoff und kräftigem Rühren eine Lösung von 238 g (1,2 Mol) 4-Phenoxybuttersäurechlorid, gelöst in 350 ml 1,2-Dichlorethan, zugetropft. Man rührt 1,5 Stunden bei 0 °C nach und läßt über Nacht auf Zimmertemperatur erwärmen. Man gibt den Ansatz unter Rühren auf eine Mischung aus 2 000 ml konzentrierte Salzsäure/2,5 kg Eis und rührt 1,5 Stunden nach. Man dekantiert die wäßrige Phase ab und befreit die organische Phase vom ungelösten Rückstand durch Filtration und wäscht gut mit 1,2-Dichlorethan nach. Die wäßrige Phase wird noch 3 - 4 Mal mit Methylenchlorid extrahiert und die vereinigten organischen Phasen mit gesättigter Natriumhydrogencarbonatlösung, Wasser und gesättigter Natriumchloridlösung gewaschen und über Natriumsulfat getrocknet. Nach Abdampfen des Lösungsmittels im Vakuum erhält man ein gelbliches Öl, das in Diethylether gelöst und von letzten Farbstoffresten durch Filtration befreit wird. Nach Eindampfen im Vakuum erhält man ein klares Öl, das bei 74 - 75 °C/0,02 Torr destilliert.
Ausbeute: 121,9 g ≙ 753 mmol (63 % d. Th.).
$^1$H-NMR (60 MHz/$CDCl_3$): δ = 7,83 - 6,88 (m, 4H); δ = 4,23 (t, 2H); δ = 2,90 (t, 2H); 2,23 (q, 2H).

**5-Hydroxy-2,3,4,5-tetrahydro-1-benzoxepin**

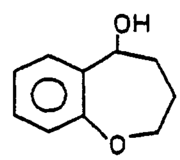

121,9 g (0,752 Mol) 2,3-Dihydro-1-benzoxepin-5(4H)-on werden in 1 400 ml Methanol gelöst und unter kräftigem Rühren portionsweise 18,5 g (0,489 Mol) Natriumborhydrid eingetragen, so daß die Temperatur unter 20 °C bleibt. Anschließend wird noch 2 Stunden nachgerührt, das Lösungsmittel im Vakuum weitgehend abdestilliert und der Rückstand in Essigester aufgenommen. Man wäscht die organische Phase mit Wasser, 1 N Kaliumhydrogensulfatlösung, Wasser, gesättigter Natriumchloridlösung und trocknet über Natriumsulfat. Man filtriert und dampft das Lösungsmittel im Vakuum ab. Nach einiger Zeit kristallisiert das Öl.
Fp.: 60 - 68,5 °C.
Ausbeute: 120 g ≙ 732 mmol (97 % d. Th.)
$^1$H-NMR (60 MHz/$CDCl_3$): δ = 7,47 - 6,78 (m, 4H); δ = 4,93 - 4,73 (m, 1H); δ = 4,13 - 4,87 (m, 2H); δ = 2,35 (brs, 1H); δ = 2,27 - 1,70 (m, 4H).

**2,3-Dihydro-1-benzoxepin**

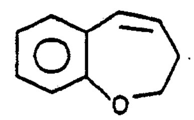

53 g (0,323 Mol) 5-Hydroxy-2,3,4,5-tetrahydro-1-benzoxepin werden mit 800 ml Toluol übergossen und unter Zugabe von 3,7 g p-Toluolsulfonsäuremonohydrat 1,5 Stunden unter Rückfluß am Wasserabscheider gekocht. Man wäscht die kalte organische Phase mit gesättigter Natriumhydrogencarbonatlösung, Wasser, Natriumchloridlösung und dampft das Lösungsmittel im Vakuum ab. Nach Kugelrohrdestillation bei 30 - 35 °C/0,008 Torr erhält man 44 g Öl.
$^1$H-NMR (60 MHz/$CDCl_3$): δ = 7,23 - 6,70 (m, 4H); δ = 6,30 (m, 1H); δ = 6,07 - 5,70 (m, 1H); δ = 4,20 (t, J = 5 Hz, 2H); δ = 2,63 (m, 2H).

**Trans-4-brom-5-hydroxy-2,3,4,5-tetrahydro-1-benzoxepin**

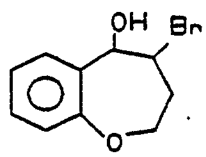

64,2 g (0,44 Mol) 2,3-Dihydro-1-benzoxepin werden in einer Mischung aus 1 800 ml Dimethylsulfoxid und 180 ml Wasser gelöst und auf 10 - 12 °C vorgekühlt. Dann werden unter starkem Rühren 156,6 g (0,88 Mol) N-Bromsuccinimid auf einmal zugegeben und die Temperatur so gehalten, daß 25 °C nicht überschritten werden. Nach 1 Stunde wird auf Eis/Wasser gegossen und 3 Mal mit Diethylether extrahiert. Die vereinigten organischen Phasen werden mit Wasser, gesättigter Natriumchloridlösung gewaschen und über Natriumsulfat getrocknet. Nach Filtration wird das Lösungsmittel im Vakuum abgedampft und das zurückbleibende gelbliche Öl in Cyclohexan kristallisiert.
Ausbeute: 63,0 g ≙ 0,259 mmol ≙ 59 % d. Th.
$^1$H-NMR (60 MHz/$CDCl_3$): δ = 7,50 - 6,85 (m, 4H); δ = 4,88 (d, J = 7 Hz, 1H); δ = 4,58 - 4,30 (m, 1H); δ = 4,23 - 4,00 (m, 2H); δ = 3,13 - 1,93 (m, 3H).

**4,5-Epoxy-2,3,4,5-tetrahydro-1-benzoxepin**

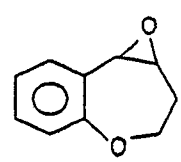

8,60 g (0,298 Mol) Natriumhydrid (80 %ige Dispersion in Mineralöl) werden unter Argon in 320 ml DMSO eingetragen und innerhalb von 2,5 Stunden bei Zimmertemperatur eine Lösung von 243 g (0,259 Mol) Trans-4-brom-5-hydroxy-2,3,4,5-tetrahydro-1-benzoxepin in 180 ml DMSO zugetropft. Nach 3 Stunden wird der Ansatz unter Rühren auf Eis/Wasser gegeben und 3 Mal mit Diethylether extrahiert. Die vereinigten organischen Phasen werden mit Wasser, gesättigter Natriumchloridlösung gewaschen, über Natriumsulfat getrocknet, filtriert und das Lösungsmittel im Vakuum eingedampft. Zurück bleiben 44 g Öl, das bei 67 - 69 °C/0,01 Torr destilliert wird.
Ausbeute 37,1 g ≙ 0,229 Mol (88 % d. Th.)
$^1$H-NMR (60 MHz/$CDCl_3$): δ = 7,50 - 6,73 (m, 4H); δ = 4,17 - 3,80 (m, 3H); δ = 3,73 - 3,53 (m, 1H); δ = 2,66 - 2,37 (m, 2H).

**2,3-Dihydro-7-nitro-1-benzoxepin-5(4H)-on**

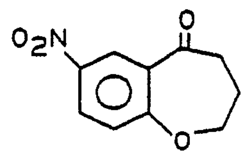

Es werden 40,5 g (0,25 Mol) 2,3 Dihydro-1-benzoxepin-5(4H)-on bei -10 °C unter Rühren in 400 ml konzentrierter Schwefelsäure eingetragen. Unter kräftigem Rühren trägt man bei -5 °C bis 0 °C langsam grobkristallines Natriumnitrat ein. Man rührt ca. 1 Stunde bei 0 °C nach, wobei das Natriumnitrat langsam in Lösung geht. Man rührt den Ansatz in Eis/Wasser ein, saugt nach ca. 30 Minuten ab und wäscht sorgfältig mit Wasser nach. Den Filterrückstand nimmt man mit Essigester auf, trennt vomW asser ab, trocknet über Natriumsulfat, filtriert und dampft im Vakuum ein. Der Rückstand wird in Essigester/Cyclohexan kristallisiert.
Smp.: 112 - 120 °C.
Ausbeute: 32,1 g ≙ 0,156 Mol (62 % d. Th.).
$^1$H-NMR (60 MHz/$CDCl_3$): δ = 8,67 (d, J = 3 Hz, 1 H); δ = 8,27 (d,d, $J_1$ = 9 Hz, $J_2$ = 3 Hz, 1H); δ = 7,20 (d, J = 9 Hz, 1H); δ = 4,38 (t, J = 7 Hz, 2H); δ = 2,95 (t,d, $J_1$ = 7 Hz, $J_2$ = 1 Hz, 2H); δ = 2,32 (m, 2H).

**5-Hydroxy-7-nitro-2,3,4,5-tetrahydro-1-benzoxepin-**

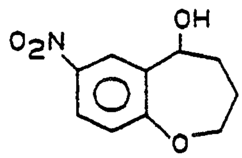

32,1 g (0,155 Mol) 2,3-Dihydro-7-nitro-1-benzoxepin5(4H)-on werden in 220 ml Methanol suspendiert und unter Rühren bei 0 °C unter Argon portionsweise mit 6,5 g (0,17 Mol) Natriumborhydrid versetzt. Danach rührt man noch ca. 30 Minuten nach und rührt den Ansatz in Eis/Wasser ein, saugt vom ausgefallenen Feststoff ab und wäscht gut mit Wasser nach. Den Feststoff nimmt man in Essigester auf, trennt vom Wasser ab, trocknet über Natriumsulfat, filtriert und dampft im Vakuum ein. Der Rückstand wird in Diisopropylether/Cyclohexan unter Zusatz von Aktivkohle kristallisiert. Smp.: 83 - 85 °C.
Ausbeute: 23 g ≙ 0,11 Mol (71 % d. Th.).
$^1$H-NMR (60 MHz/$CDCl_3$): δ = 8,43 (d, J = 3 Hz, 1H); δ = 8,10 (d,d, $J_1$ = 9 Hz, $J_2$ = 3 Hz, 1 H); δ = 7,10 (d, J = 9 Hz, 1H); δ = 5,23 - 4,83 (m, 1H); δ = 4,53 - 3,60 (m, 2H); δ = 2,40 - 1,73 (m, 5H).

**2,3-Dihydro-7-nitro-1-benzoxepin**

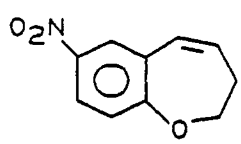

25,1 g (0,12 Mol) 5-Hydroxy-7-nitro-2,3,4,5-tetrahydro-1-benzoxepin werden mit 300 ml Toluol übergossen und unter Zusatz von 1,5 g p-Toluolsulfonsäure 2 Stunden unter Rückfluß am Wasserabscheider gekocht. Die kalte Reaktionslösung wäscht man mit gesättigter Natriumhydrogencarbonatlösung, Wasser, gesättigter Natriumchloridlösung, trocknet über Natriumsulfat, filtriert und dampft das Lösungsmittel im Vakuum ab. Zurück bleibt ein Feststoff vom Smp. 94 - 95,5 °C.
Ausbeute: 22 g ≙ 0,115 Mol (96 % d. Th.).

**Trans-4-brom-5-hydroxy-7-nitro-2,3,4,5-tetrahydro-1-benzoxepin**

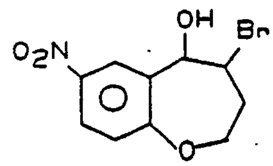

22,9 g (0,12 Mol) 2,3-Dihydro-7-nitro-1-benzoxepin werden in einer Mischung aus 460 ml Dimethylsulfoxid und 46 ml Wasser auf ca. 12 °C vorgekühlt und unter kräftigem Rühren 42,7 g (0,24 Mol) N-Bromsuccinimid zugesetzt, wobei man die Temperatur unter 25 °C hält. Man rührt noch ca. 30 Minuten bei Zimmertemperatur nach und rührt den Ansatz auf Eis/Wasser und extrahiert mit Diethylether. Die vereinigten organischen Phasen wäscht man mit Wasser, gesättigter Natriumchloridlösung, trocknet über Natriumsulfat, filtriert und dampft das Lösungsmittel im Vakuum ab. Der feste Rückstand wird mit Cyclohexan verrieben und abgesaugt.
Ausbeute: 32,2 g (0,112 Mol ≙ 93 % d. Th.).
Smp.: 105 - 106,5 °C.

**4,5-Epoxy-7-nitro-2,3,4,5-tetrahydro-benzoxepin**

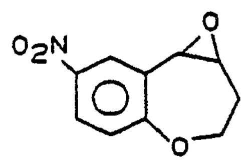

2,65 g (0,115 Mol) metallisches Natrium werden in 220 ml Methanol gelöst und unter Rühren eine Lösung von 31,7 g (0,11 Mol) Trans-4-brom-5-hydroxy-7-nitro-2,3,4,5-tetrahydro-1-benzoxepin in 230 ml Methanol bei Zimmertemperatur zugetropft. Nach 2,5 Stunden saugt man vom ausgefallenen Feststoff ab, wäscht mit Methanol nach und dampft das Filtrat im Vakuum ein. Zurück bleibt ein Feststoff vom Schmelzpunkt 121 - 123 °C.
Ausbeute: 17,9 g ≙ 86,5 mmol (79 % d. Th.).
$^1$H-NMR (60 MHz/$CDCl_3$): δ = 8,48 (d, J = 3 Hz, 1H); δ = 8,07 (d,d, J = 9 Hz, 1H); δ = 7,02 (d, J = 9 Hz, 1H); δ = 4,33 - 3,93 (m, 3H); 3,83 - 3,62 (m, 1H); δ = 2,67 - 2,37 (m, 2H).

**7-Brom-2,3-tetrahydro-1-benzoxepin**

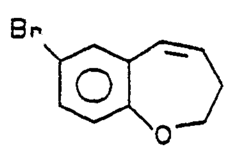

125 g (0,762 Mol) 5-Hydroxy-2,3,4,5-tetrahydro-1-benzoxepin werden in 938 ml Eisessig gelöst, auf 10 °C gekühlt und unter kräftigem Rühren innerhalb von 30 Minuten 135,3 g (0,760 Mol) N-Bromsuccinimid eingetragen, wobei die Temperatur unter 20 °C gehalten wird. Man rührt 3 Stunden bei 20 °C nach, gießt den Ansatz unter kräftigem Rühren auf 3 l Eiswasser, saugt ab und wäscht gut mit Wasser nach. Der Rückstand wird nochmals gut mit Wasser verrieben und abgesaugt. Das feuchte Rohprodukt wird mit 1 700 ml Toluol übergossen und unter Zusatz von 2,00 g p-Toluolsulfonsäuremonohydrat am Wasserabscheider 3 Stunden unter Rückfluß erhitzt. Das Lösungsmittel wird im Vakuum abgedampft und über Kieselgel filtriert. Mobile Phase ist zunächst Cyclohexan, dann Cyclohexan/Methyl-tert.-butylether (200 : 1). Nach Eindampfen aller Fraktionen im Vakuum bleibt ein helles Öl zurück.

Ausbeute: 126 g ≙ 0,56 Mol (73 % d. Th.).
$^1$H-NMR ($CDCl_3$/60 MHz): δ = 7,23 (sbr, 1H); δ = 7,13 (d,d, $J_1$ = 9 Hz, $J_2$ = 3 Hz, 1H); δ = 7,77 (dbr, J = 9 Hz, 1H); δ = 6,37 - 5,73 (m, 2H); δ = 4,18 (t, J = 5 Hz, 2H); δ = 2,8 - 2,43 (m, 2H).

**2,3-Dihydro-7-phenylthio-1-benzoxepin**

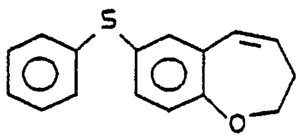

20 g (89,3 mmol) 7-Brom-2,3-dihydro-1-benzoxepin werden in 180 ml trockenem Tetrahydrofuran gelöst und unter Argon bei -78° C 105 ml (178,6 mmol) tert.-Butyllithium innerhalb von 1 Stunde zugetropft. Man rührt 4,5 Stunden bei -78° C nach und tropft dann bei dieser Temperatur 19,6 g (89 mmol) Diphenyldisulfid, gelöst in 90 ml getrocknetem Tetrahydrofuran, zu. Man läßt die Temperatur über ca. 15 Stunden auf 0° C ansteigen, hydrolysiert mit kaltem Wasser und extrahiert mehrmals mit Diethylether. Die vereinigten organischen Extrakte werden mit kalter 5 %iger Kaliumhydroxidlösung, Wasser und gesättigter Natriumchloridlösung gewaschen. Man trocknet über Natriumsulfat, filtriert und dampft das Lösungsmittel im Vakuum ab. Das zurückbleibende braune Öl wird mit Cyclohexan über eine kurze Kieselgelsäule (200 g) filtriert. Nach Eindampfen aller Fraktionen bleiben 13,6 g ≙ 53,3 mmol (60 % d. Th.) helles Öl zurück.
$^1$H-NMR ($CDCl_3$/60 MHz): δ = 7,80 - 6,77 (m, 8H); δ = 6,53 - 5,77 (m, 2H); δ = 4,25 (t, J = 5 Hz, 2H); δ = 2,83 - 2,47 (m, 2H).

**2,3-Dihydro-7-phenylsulfinyl-1-benzoxepin**

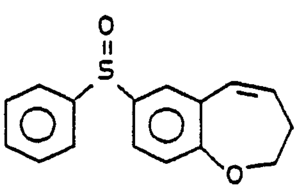

6,00 g (23,6 mmol) 2,3-Dihydro-7 phenylthio-1-benzoxepin werden bei 15 - 20° C in 59 ml Eisessig vorgelegt und bei dieser Temperatur 5 ml wäßrige Wasserstoffperoxidlösung (30 %ig) zugetropft. Man rührt ca. 2 Stunden bei dieser Temperatur nach, bis alles Edukt abreagiert hat (DC-Kontrolle). Dann rührt man den Ansatz in Natriumhydrogencarbonat/Eiswasser und extrahiert mehrfach mit Diethylether. Man wäscht die vereinigten Etherphasen mit Wasser, gesättigter Natriumchloridlösung, trocknet über Natriumsulfat, filtriert und dampft das Lösungsmittel im Vakuum ab. Zurück bleiben 6,2 g Öl, das ohne weitere Reinigung umgesetzt wird.

**Trans-4-brom-5-hydroxy-7-phenylsulfinyl-2,3,4,5-tetrahydro-1-benzoxepin**

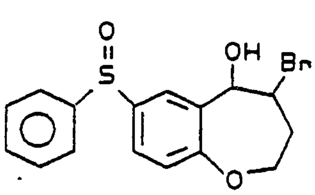

6,20 g des rohen 2,3-Dihydro-7-phenylsulfinyl-1-benzoxepins werden in einer Mischung aus 88 ml DMSO und 8,8 ml Wasser gelöst und auf ca. 10 - 12° C gekühlt. Dann werden bei dieser Temperatur auf

einmal 8,2 g N-Bromsuccinimid unter kräftigem Rühren zugesetzt und dabei die Temperatur unter 25 °C gehalten. Man rührt ca. 1 Stunde bei 20 °C nach, rührt den Ansatz in Eiswasser ein und extrahiert mehrmals mit Ether. Die vereinigten Etherphasen werden mit Wasser, gesättigter Natriumchloridlösung gewaschen und über Natriumsulfat getrocknet. Nach Filtration und Abdampfen des Lösungsmittels im Vakuum wird der Rückstand mit Methyl-tert.-butylether verrieben und abgesaugt. Man erhält weiße Kristalle vom Smp. 147 - 149 °C.

$^1$H-NMR (270 MHz/d$^6$-DMSO): δ = 7,75 - 7,67 (m, 3H); δ = 7,61 - 7,46 (m, 4H); δ = 7,09 (d, J = 8 Hz, 1H); δ = 6,12 (d, J = 5 Hz, 1H); δ = 4,85 (brt, J = 5 Hz, 1H); δ = 4,51 (m, 1H); δ = 4,12 (t, J = 4 Hz, 2H); δ = 2,78 - 2,61 (m, 1H); δ = 2,19 - 2,04 (m, 1H).

**7-Brom-5-hydroxy-2,3,4,5-tetrahydro-1-benzoxepin**

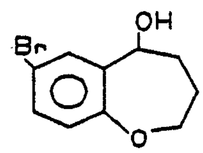

104,9 g (0,64 Mol) 2,3-Dihydro-1-benzoxepin-5(4H)-on werden bei Zimmertemperatur in 780 ml Eisessig gelöst, und danach werden bei 10 °C portionsweise unter starkem Rühren 113,9 g (0,64 Mol) N-Bromsuccinimid eingetragen, so daß die Temperatur 18 - 20 °C nicht überschreitet (ca. 30 min). Nach ca. 4 Stunden (DC-Kontrolle) ist die Umsetzung beendet und man gibt den Ansatz unter kräftigem Rühren auf 3 l Eiswasser und rührt 30 Minuten nach. Nach einiger Zeit kristallisiert der zunächst schmierig anfallende Feststoff durch. Der Feststoff wird abgesaugt, mit ca. 5 l Wasser nachgewaschen. Man trocknet im Vakuum über Calciumchlorid und kristallisiert in Cyclohexan.

Ausbeute: 117 g, Smp. 86 - 88 °C.

**7-Formyl-5-hydroxy-2,3,4,5-tetrahydro-1-benzoxepin**

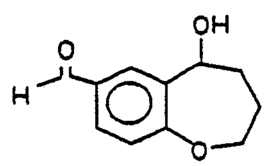

In eine Lösung von 48,6 g (0,2 Mol) 7-Brom-5-hydroxy-2,3,4,5-tetrahydro-1-benzoxepin in 550 ml absolutem THF wird unter Argon und Rühren bei -90 °C bis -100 °C langsam 254 ml (0,6 Mol) einer 1,7-molaren Lösung von tert.-Butyllithium in n-Pentan zugetropft. Man rührt anschließend 1 Stunde bei -78 °C nach und tropft dann bei dieser Temperatur eine Lösung von 24 ml (0,24 Mol) N-Formylmorpholin in 180 ml absolutem THF unter kräftigem Rühren zu. Man rührt noch 1 Stunde bei -78 °C nach, läßt anschließend auf 0 °C erwärmen, verdünnt mit Diethylether und hydrolysiert mit Eiswasser. Nach mehrmaligem Extrahieren mit Diethylether werden die vereinigten organischen Extrakte mit Wasser und gesättigter Kochsalzlösung gewaschen, über Natriumsulfat getrocknet, und das Lösungsmittel wird im Vakuum abgezogen. Man erhält 40,7 g gelbliches Öl, das ohne Reinigung weiter umgesetzt wird.

**7-Cyano-5-hydroxy-2,3,4,5-tetrahydro-1-benzoxepin**

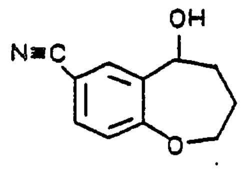

38 g des oben isolierten Öls werden in 210 ml Wasser bei Raumtemperatur mit einer Lösung von 27 g Hydroxylamin-O-sulfonsäure in 90 ml Wasser unter kräftigem Rühren versetzt. Man rührt 0,5 Stunden bei Zimmertemperatur und erhitzt anschließend 1 Stunde auf 60° C. Der Ansatz wird anschließend mit eiskalter 1N-Kaliumhydrogencarbonatlösung hydrolysiert und mit Essigester mehrmals extrahiert. Die vereinigten organischen Extrakte werden mit Wasser und gesättigter Natriumchloridlösung gewaschen, über Natriumsulfat getrocknet, und das Lösungsmittel wird im Vakuum abgedampft. Zurück bleiben 34 g gelbliches Öl, das an Kieselgel chromatographiert wird (Methyl-tert.-butylether/Cyclohexan 1 : 2). Die Substanz kristallisiert beim Eindampfen der sauberen Fraktionen.
Ausbeute: 19,1 g; Smp. 85 - 86,5° C.

**7-Cyano-2,3-dihydro-1-benzoxepin**

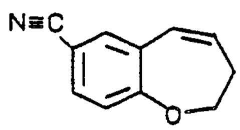

18,9 g (0,1 Mol) 7-Cyano-5-hydroxy-2,3,4,5-tetrahydro-1-benzoxepin werden mit 0,3 g p-Toluolsulfonsäure-monohydrat in Toluol 1,5 Stunden am Wasserabscheider erhitzt. Man wäscht nach Erkalten der Lösung mit 1N-Kaliumhydrogencarbonatlösung, Wasser, gesättigter Natriumchloridlösung und dampft nach Trocknung über Natriumsulfat das Lösungsmittel im Vakuum ein. Man erhält 16,3 g Feststoff vom Smp. 69 - 71° C.

**Trans-4-brom-7-cyan-5-hydroxy-2,3,4,5-tetrahydro-1-benzoxepin**

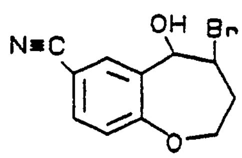

Zu einer Lösung von 16,25 g (95 mmol) 7-Cyano-2,3-dihydro-1-benzoxepin in 363 ml Dimethylsulfoxid/Wasser 10 : 1 werden unter kräftigem Rühren bei 12 - 15° C 35,5 g (200 mmol) N-Bromsuccinimid auf einmal zugesetzt. Die Temperatur wird mit Hilfe eines Kältebads so gehalten, daß 30° C nicht überschritten werden. Man rührt nach Abklingen der exothermen Reaktion noch 0,5 Stunden bei Zimmertemperatur nach und hydrolysiert den Ansatz mit Eis/Wasser. Man extrahiert 3 mal mit Essigester, wäscht die organischen Extrakte mit Wasser, gesättigter Natriumchloridlösung und trocknet über Natriumsulfat. Nach Abdampfen des Lösungsmittels im Vakuum wird der Rückstand mit Cyclohexan verrührt und der Feststoff abgesaugt.
Ausbeute: 22,6 g; Smp. 126 - 128° C.
$^1$H-NMR (60 MHz/$CDCl_3$): $\delta$ = 7,87 (d, J = 2 Hz, 1H); $\delta$ = 7,58 (d, d, $J_1$ = 8 Hz, $J_2$ = 2 Hz, 1H); $\delta$ = 7,1 (d, J = 8 Hz, 1H); $\delta$ = 4,95 (m, 1H); $\delta$ = 4,60 - 3,83 (m, 3H); $\delta$ = 3,1 - 2,0 (m, 3 H).

**7-Cyano-4,5-epoxy-2,3,4,5-tetrahydro-1-benzoxepin**

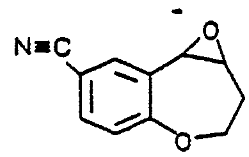

Man tropft eine Lösung von 22,5 g (84 mmol) Trans-4-brom-7-cyan-5-hydroxy-2,3,4,5-tetrahydro-1-benzoxepin in 80 ml DMSO unter Argon zu einer gerührten Suspension von 4,44 g (92,4 mmol) Natriumhydrid (55 %ig in Öl) in 120 ml DMSO. Man hält hierbei die Temperatur bei 26 - 31 °C und rührt noch 1 Stunde bei Zimmertemperatur nach. Man hydrolysiert den Ansatz vorsichtig mit Eis/Wasser, rührt ca. 20 min nach, saugt den ausgefallenen Feststoff ab und wäscht gut mit Wasser nach. Der Feststoff wird mit Cyclohexan verrührt, abgesaugt, gut mit Cyclohexan nachgewaschen und bei 60 °C im Vakuum getrocknet.

Ausbeute: 14,1 g vom Smp. 118 - 119 °C.

$^1$H-NMR (60 MHz/$CDCl_3$): $\delta$ = 7,80 (d, J = 2 Hz, 1H); $\delta$ = 7,53 (d, d, $J_1$ = 8 Hz, $J_2$ = 2Hz, 1H); $\delta$ = 6,97 (d, J = 8 Hz, 1H); $\delta$ = 4,12 (m, 2H); $\delta$ = 3,77 (m, 2H); $\delta$ = 2,55 (m, 2H).

## Ansprüche

1. 2,3,4,5-Tetrahydro-1-benzoxepine der Formel I

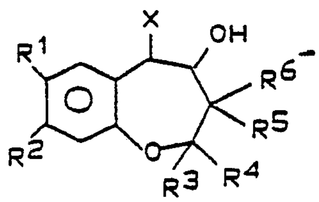 (I),

in welcher

$R^1$ für H, ($C_1$-$C_4$)-Alkyl, OH, ($C_1$-$C_4$)-Alkoxy, Halogen, CN, $NO_2$, CO-($C_1$-$C_4$)-Alkyl, -CH(OH)-($C_1$-$C_4$)-Alkyl, CO-Ar, -CH(OH)-Ar, COOH, Carboxy-($C_1$-$C_4$)-alkyl, ($C_1$-$C_4$)-Alkyl-$SO_r$-, Ar$SO_r$-, wobei r für 0, 1, 2 und Ar für ein aromatisches oder heteroaromatisches System steht, das unsubstituiert oder durch 1 bis 3 gleiche oder verschiedene Reste ($C_1$-$C_4$)-Alkyl, ($C_1$-$C_4$)-Alkoxy, Halogen, CN, $NO_2$ substituiert ist, steht,

$R^2$ für H, OH, ($C_1$-$C_4$)-Alkoxy, ($C_1$-$C_4$)-Alkyl steht,

$R^3$,$R^4$,$R^5$,$R^6$ gleich oder verschieden sind und für H oder ($C_1$-$C_4$)-Alkyl stehen und

X in der Bedeutung von α) oder β) steht,

α) 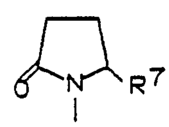

β) 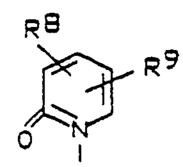 ,

wobei $R^7$ H oder Methyl, $R^8$ und $R^9$ gleich oder verschieden sind und für H, ($C_1$-$C_2$)-Alkyl, Halogen, Nitro oder CN stehen.

2. Verbindung I nach Anspruch 1, dadurch gekennzeichnet, daß $R^1$ für H, Halogen, CN, Nitro, Phenylsulfinyl, Phenylsulfonyl und Benzoyl steht, wobei die Phenylreste in der obengenannten Bedeutung substituiert sind und $R^2$, $R^3$, $R^4$, $R^5$, $R^6$ und X wie im Anspruch 1 definiert sind.

3. Verbindung I nach Anspruch 1, dadurch gekennzeichnet, daß $R^1$ für H, Halogen, CN, Nitro, Phenylsulfinyl, Phenylsulfonyl und Benzoyl steht, wobei die Phenylreste unsubstituiert oder durch 1 bis 2 gleiche oder verschiedene Halogenatome substituiert sind, $R^2$ H bedeutet, $R^3$, $R^4$, $R^5$, $R^6$ und X wie in Anspruch 1 definiert sind.

4. Verbindung I nach Anspruch 1, dadurch gekennzeichnet, daß $R^1$ für H, CN, Nitro, Phenylsulfinyl, Phenylsulfonyl und Benzoyl steht, wobei die Phenylreste unsubstituiert oder durch 1 bis 2 gleiche oder verschiedene Halogenatome substituiert sind, $R^2$, $R^3$, $R^4$, $R^5$ und $R^6$ für ein Wasserstoffatom und X in der Bedeutung von ß steht, wobei $R^8$ und $R^9$ die in Anspruch 1 angegebenen Definitionen haben.

5. Verbindung I nach Anspruch 1, dadurch gekennzeichnet, daß $R^1$ für H, CN, Nitro, Phenylsulfinyl, Phenylsulfonyl und Benzoyl steht, wobei die Phenylreste unsubstituiert oder durch ein Halogenatom substituiert sind, $R^2$, $R^3$, $R^4$, $R^5$, $R^6$ für ein Wasserstoffatom und X in der Bedeutung von $\alpha$ steht, wobei $R^7$ wie oben definiert ist.

6. Verfahren zum Herstellen einer Verbindung I nach Anspruch 1, dadurch gekennzeichnet, daß man

a) Verbindungen der Formel II

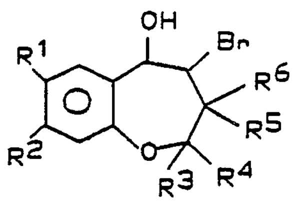

(II),

in denen $R^1$ bis $R^6$ wie in Anspruch 1 definiert sind, umsetzt mit Verbindungen der Formel III oder IV

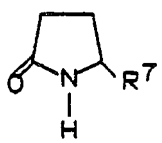

(III)

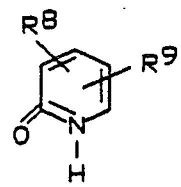

(IV)

oder daß man

b) Verbindungen der Formel V

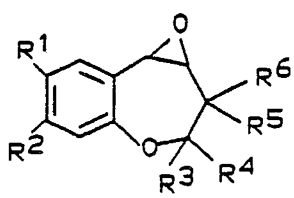

(V),

in denen $R^1$ bis $R^6$ wie in Anspruch 1 definiert sind, umsetzt mit Verbindungen der Formel III oder IV

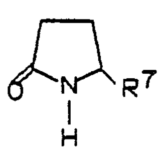

(III)

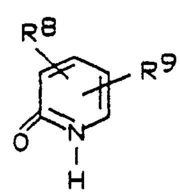

(IV),

oder daß man

c) Verbindungen der Formel II

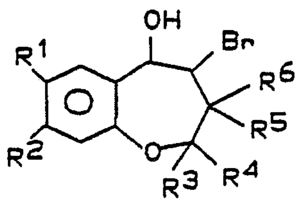 (II),

in denen $R^1$ bis $R^6$ wie in Anspruch 1 definiert sind, umsetzt mit Verbindungen der Formel VI und VII

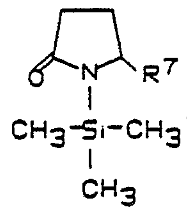 (VI)

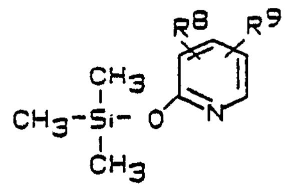 (VII),

oder daß man

d) Verbindungen der Formel V

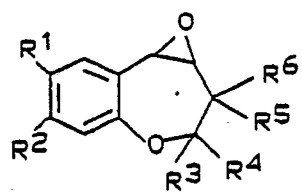 (V),

in denen $R^1$ bis $R^6$ wie in Anspruch 1 definiert sind, umsetzt mit Verbindungen der Formel VI und VII

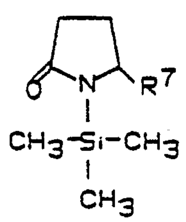 (VI)

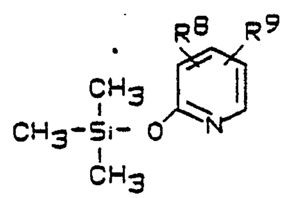 (VII).

7. Verwendung einer Verbindung I nach Anspruch 1 zur Herstellung eines Arzneimittels zur Behandlung der Hypertonie, der Angina pectoris, der Herzinsuffizienz, von peripheren Durchblutungsstörungen, von Darmmotilitätsstörungen, vorzeitiger Wehentätigkeit, Obstruktionen der Atemwege, der ableitenden Harn- oder Gallenwege oder von Krampfzuständen.

8. Arzneimittel zur Behandlung der Hypertonie, der Angina pectoris, der Herzinsuffizienz, von peripheren Durchblutungsstörungen, von Darmmotilitätsstörungen, vorzeitiger Wehentätigkeit, Obstruktionen der Atemwege, der ableitenden Harn- oder Gallenwege oder von Krampfzuständen, gekennzeichnet durch einen wirksamen Gehalt einer Verbindung I nach Anspruch 1.

9. Verwendung einer Verbindung I zur Behandlung von Herz-Kreislaufstörungen.

10. Verwendung einer Verbindung I zur Behandlung von Spasmen der ableitenden Harnwege.

11. Verwendung einer Verbindung I zur Behandlung obstruktiver Atemwegserkrankungen.

12. Verwendung einer Verbindung I als Spasmolytikum.

Patentansprüche für folgende Vertragsstaaten: ES, GR

1. Verfahren zum Herstellen einer Verbindung I

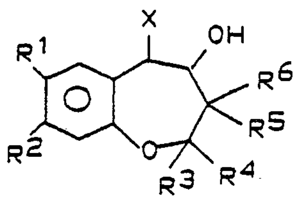

(I),

in welcher

$R^1$ für H, $(C_1\text{-}C_4)$-Alkyl, OH, $(C_1\text{-}C_4)$-Alkoxy, Halogen, CN, $NO_2$, CO-$(C_1\text{-}C_4)$-Alkyl, $-\overset{OH}{CH}-(C_1\text{-}C_4)$-Alkyl, CO-Ar, $-\overset{OH}{CH}$-Ar, COOH, Carboxy-$(C_1\text{-}C_4)$-alkyl, $(C_1\text{-}C_4)$-Alkyl-$SO_r$-, Ar$SO_r$-, wobei r für 0, 1, 2 und Ar für ein aromatisches oder heteroaromatisches System steht, das unsubstituiert oder durch 1 bis 3 gleiche oder verschiedene Reste $(C_1\text{-}C_4)$-Alkyl, $(C_1\text{-}C_4)$-Alkoxy, Halogen, CN, $NO_2$ substituiert ist, steht,

$R^2$ für H, OH, $(C_1\text{-}C_4)$-Alkoxy, $(C_1\text{-}C_4)$-Alkyl steht,

$R^3,R^4,R^5,R^6$ gleich oder verschieden sind und für H oder $(C_1\text{-}C_4)$-Alkyl stehen und

X in der Bedeutung von α) oder ß) steht,

α) 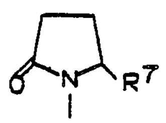

ß) 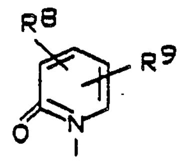

,

wobei $R^7$ H oder Methyl, $R^8$ und $R^9$ gleich oder verschieden sind und für H, $(C_1\text{-}C_2)$-Alkyl, Halogen, Nitro oder CN stehen,

dadurch gekennzeichnet, daßm an

a) Verbindungen der Formel II

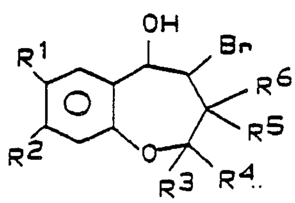

(II),

in denen $R^1$ bis $R^6$ wie oben definiert sind,

umsetzt mit Verbindungen der Formel III oder IV

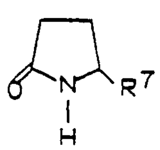

(III)

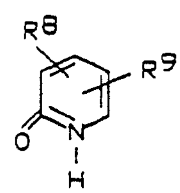

(IV)

oder daß man

b) Verbindungen der Formel V

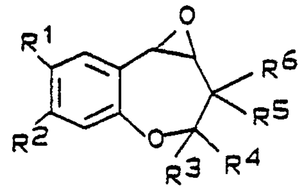

(V),

in denen $R^1$ bis $R^6$ wie oben definiert sind, umsetzt mit Verbindungen der Formel III oder IV

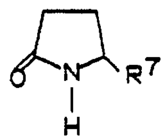

(III)

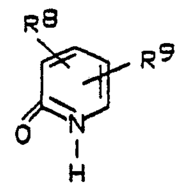

(IV),

oder daß man c) Verbindungen der Formel II

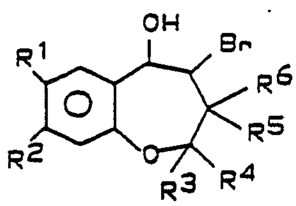

(II),

in denen $R^1$ bis $R^6$ wie oben definiert sind, umsetzt mit Verbindungen der Formel VI und VII

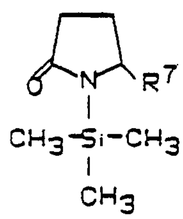

(VI)

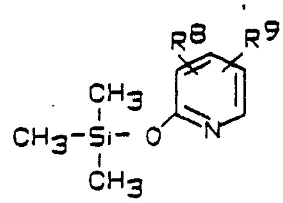

(VII),

oder daß man

d) Verbindungen der Formel V

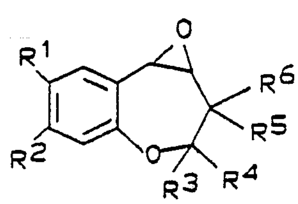

(V),

in denen $R^1$ bis $R^6$ wie oben definiert sind, umsetzt mit Verbindungen der Formel VI und VII

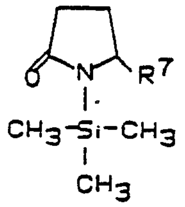

(VI)

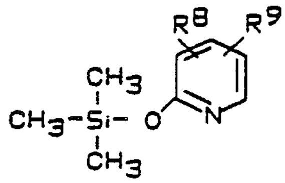

(VII).

2. Verfahren nach Anspruch 1, dadurch gekennzeichnet, daß $R^1$ für H, Halogen, CN, Nitro, Phenylsulfinyl, Phenylsulfonyl und Benzoyl steht, wobei die Phenylreste in der obengenannten Bedeutung substituiert sind und $R^2$, $R^3$, $R^4$, $R^5$, $R^6$ und X wie im Anspruch 1 definiert sind.

3. Verfahren nach Anspruch 1, dadurch gekennzeichnet, daß $R^1$ für H, Halogen, CN, Nitro, Phenylsulfinyl, Phenylsulfonyl und Benzoyl steht, wobei die Phenylreste unsubstituiert oder durch 1 bis 2 gleiche oder verschiedene Halogenatome substituiert sind, $R^2$ H bedeutet, $R^3$, $R^4$, $R^5$ $R^6$ und X wie in Anspruch 1 definiert sind.

4. Verfahren nach Anspruch 1, dadurch gekennzeichnet, daß $R^1$ für H, CN, Nitro, Phenylsulfinyl, Phenylsulfonyl und Benzoyl steht, wobei die Phenylreste unsubstituiert oder durch 1 bis 2 gleiche oder verschiedene Halogenatome substituiert sind, $R^2$, $R^3$, $R^4$, $R^5$ und $R^6$ für ein Wasserstoffatom und X in der Bedeutung von 3 steht, wobei $R^8$ und $R^9$ die in Anspruch 1 abgegebenen Definitionen haben.

5. Verfahren nach Anspruch 1, dadurch gekennzeichnet, daß $R^1$ für H, CN, Nitro, Phenylsulfinyl, Phenylsulfonyl und Benzoyl steht, wobei die Phenylrest unsubstituiert oder durch ein Halogenatom substituiert sind, $R^2$, $R^3$, $R^4$, $R^5$, $R^6$ für ein Wasserstoffatom und X in der Bedeutung von $\alpha$ steht, wobei $R^7$ wie oben definiert ist.

6. Verfahren zum Herstellen eines Arzneimittels zur Behandlung der Hypertonie, der Angina pectoris, der Herzinsuffizienz, von peripheren Durchblutungsstörungen, von Darmmotilitätsstörungen, vorzeitiger Wehentätigkeit, Obstruktionen der Atemwege, der ableitenden Harn- oder Gallenwege oder von Krampfzuständen, dadurch gekennzeichnet, daß man eine wirksame Menge einer nach Anspruch 1 erhaltenen Verbindung mit den üblichen Zuschlagstoffen vermischt und in eine geeignete Darreichungsform bringt.

7. Verwendung einer Verbindung I nach Anspruch 1 zur Herstellung eines Arzneimittels zur Behandlung der Hypertonie, der Angina pectoris, der Herzinsuffizienz, von peripheren Durchblutungsstörungen, von Darmmotilitätsstörungen, vorzeitiger Wehentätigkeit, Obstruktionen der Atemwege, der ableitenden Harn- oder Gallenwege oder von Krampfzuständen.

8. Verwendung einer Verbindung I zur Behandlung von Herz-Kreislaufstörungen.

9. Verwendung einer Verbindung I zur Behandlung von Spasmen der ableitenden Harnwege.

10. Verwendung einer Verbindung I zur Behandlung obstruktiver Atemwegserkrankungen.

11. Verwendung einer Verbindung I als Spasmolytikum.

Europäisches Patentamt

**EUROPÄISCHER TEILRECHERCHENBERICHT,**
der nach Regel 45 des Europäischen Patentübereinkommens für das weitere Verfahren als europäischer Recherchenbericht gilt

Nummer der Anmeldung

EP 89116833.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A1 - 0 116 372 (MERRELL) * Beispiel 1 * | 1 | C 07 D 405/04<br>A 61 K 31/40<br>A 61 K 31/44 |
| A | CHEMICAL ABSTRACTS, Band 109, Nr. 11, 2. September 1988, Columbus, Ohio, USA CARLESS, HOWARD A.J. et al. "Intramolecular hydrogen abstraction in ketone photochemistry: the first examples of ζ-hydrogen abstraction" Seite 622, Spalte 1, Zusammenfassung-Nr. 92 063t & J. Chem. Soc., Chem. Commun. 1987, (21), 1673-4 | 1 | |
| A | CHEMICAL ABSTRACTS, Band 104, Nr. 23, 9. Juni 1986, Columbus, Ohio, USA KAUPMANN, WILHELM et al. "Synthesis of 3-amino-2,3,4, 5-tetrahydro-1-benzoxepin-5- | 1 | RECHERCHIERTE SACHGEBIETE (Int. Cl.4)<br>C 07 D 405/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-8
Unvollständig recherchierte Patentansprüche: –
Nicht recherchierte Patentansprüche: 9-12
Grund für die Beschränkung der Recherche:

(Art. 52(4) EPÜ; Verfahren zur therapeutischen Behandlung des menschlichen oder tierischen Körpers)

| Recherchenort | Abschlußdatum der Recherche | Prüfer |
|---|---|---|
| WIEN | 27-11-1989 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03.82

Europäisches Patentamt

# EUROPÄISCHER TEILRECHERCHENBERICHT

Nummer der Anmeldung

-2-

EP 89116833.8

| EINSCHLÄGIGE DOKUMENTE | | | KLASSIFIKATION DER ANMELDUNG (Int. Cl.4) |
|---|---|---|---|
| Kategorie | Kennzeichnung des Dokuments mit Angabe, soweit erforderlich, der maßgeblichen Teile | betrifft Anspruch | |
| | -ols and their action on gastric motility"<br>Seite 741, Spalte 2, Zusammenfassung-Nr. 207 126g<br>& Eur. J. Med. Chem.--Chim. Ther. 1985, 20(3), 207-12<br>---- | | |
| | | | RECHERCHIERTE SACHGEBIETE (Int. Cl.4) |